(19) Europäisches Patentamt
European Patent Office
Office européen des brevets

(11) **EP 2 437 726 B1**

(12) **EUROPEAN PATENT SPECIFICATION**

(45) Date of publication and mention
of the grant of the patent:
**16.05.2018 Bulletin 2018/20**

(51) Int Cl.:
*A61K 9/127* (2006.01)   *A61K 9/00* (2006.01)
*A61K 47/26* (2006.01)   *A61K 9/10* (2006.01)
*A61K 9/107* (2006.01)   *A61K 9/12* (2006.01)
*A61K 47/24* (2006.01)   *A61K 47/10* (2017.01)
*A61K 9/06* (2006.01)   *A61P 19/02* (2006.01)

(21) Application number: **10742250.3**

(22) Date of filing: **03.06.2010**

(86) International application number:
**PCT/IB2010/001557**

(87) International publication number:
**WO 2010/140061 (09.12.2010 Gazette 2010/49)**

(54) **FORMULATIONS FOR THE TREATMENT OF DEEP TISSUE PAIN**

FORMULIERUNGEN ZUR BEHANDLUNG VON TIEFEM GEWEBESCHMERZ

FORMULATIONS DESTINÉES AU TRAITEMENT DE LA DOULEUR DE TISSUS PROFONDS

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(30) Priority: **01.04.2010 US 320148 P
03.06.2009 US 183956 P
16.03.2010 US 314478 P**

(43) Date of publication of application:
**11.04.2012 Bulletin 2012/15**

(73) Proprietor: **Sequessome Technology Holdings
Limited
Valletta VLT 1436 (MT)**

(72) Inventors:
• **MAYO, John Charles
London WC1B 3RA (GB)**
• **ILIFFE, George Langton
London WC1B 3RA (GB)**
• **VIERL, Ulrich
82194 Gröbenzell (DE)**
• **ROTHER, Matthias
82166 Gräfelfing (DE)**

(74) Representative: **Care, Alison et al
Kilburn & Strode LLP
Lacon London
84 Theobalds Road
London WC1X 8NL (GB)**

(56) References cited:
EP-B1- 1 551 370          WO-A1-00/12060
WO-A2-2006/086992     US-A- 5 853 753
US-A- 6 165 500          US-A1- 2003 064 948
US-A1- 2007 224 256    US-A1- 2007 238 708

• Bayer: "Bepanthen - Spray mousse
rafraichissant (patient information leaflet)", , 10
December 2007 (2007-12-10), XP002636839,
[retrieved on 2011-05-10]
• TREEDE I. ET AL.: "Anti-inflammatory effects of
phosphatidylcholine.", THE JOURNAL OF
BIOLOGICAL CHEMISTRY, vol. 282, no. 37, 14
September 2007 (2007-09-14), pages
27155-27164, XP002636873, DOI:
10.1074/jbc.M/04408200
• BARTHEL H R ET AL: "Randomized Controlled
Trial of Diclofenac Sodium Gel in Knee
Osteoarthritis", SEMINARS IN ARTHRITIS AND
RHEUMATISM, ELSEVIER, AMSTERDAM, NL,
vol. 39, no. 3, 1 December 2009 (2009-12-01),
pages 203-212, XP026774029, ISSN: 0049-0172,
DOI: 10.1016/J.SEMARTHRIT.2009.09.002
[retrieved on 2009-11-22]

Remarks:
     The file contains technical information submitted after
     the application was filed and not included in this
     specification

Remarks:
     The file contains technical information submitted after
     the application was filed and not included in this
     specification

**Description**

**1. FIELD OF INVENTION**

The present invention relates to formulations of one or more phospholipids and one or more non-ionic surfactants, being free of any non-lipid, non-surfactant pharmaceutically active agent, wherein such formulations have deformable properties and are also referred to as "deformasomes," and to the use of such formulations for the treatment of pain associated with osteoarthritis, wherein the formulation is topically applied.

**2. BACKGROUND**

U.S. Patent No. 6,165,500 to Cevc describes a "preparation for the application of agents ... provided with membrane-like structures consisting of one or several layers of amphiphilic molecules, or an amphiphilic carrier substance, in particular for transporting the agent into and through natural barriers such as skin and similar materials." Abstract. These transfersomes "consist of one or several components[, m]ost commonly a mixture of basic substances, one or several edge-active substances, and agents []." Col. 5, lines 28-30. According to U.S. Patent No. 6,165,500, "[l]ipids and other amphiphiles are best suited basic substances; surfactants or suitable solvents are the best choice from the point of view of edge-active substances[, and a]ll of these can be mixed with agents in certain proportions depending both on the choice of the starting substances and on their absolute concentration." Col. 5, lines 30-35.

U.S. Patent Application Publication No. US 2004/0071767 to Cevc et al. describes "formulations of nonsteroidal anti-inflammatory drugs (NSAIDs) based on complex aggregates with at least three amphiphatic components suspended in a ... pharmaceutically acceptable ... medium." Abstract. "One of these components is capable of forming stable, large bilayer membranes on its own. The other at least two amphiphatic components, including an NSAID, tend to destabilise such membranes." Paragraph [0002].

U.S. Patent Application Publication No. US 2004/0105881 to Cevc et al. describes extended surface aggregates, "suspendable in a suitable liquid medium and comprising at least three amphiphats (amphiphatic components) and being capable to improve the transport of actives through semi-permeable barriers, such as the skin, especially for the non-invasive drug application in vivo by means of barrier penetration by such aggregates." Paragraph [0002]. "The three amphiphats include at least one membrane forming compound (MFC), which can form the membrane of [the aggregates], and at least two membrane destabilising compounds ($MDC_1$ and $MDC_2$) differentiated by their capability of forming smaller aggregates (with no extended surfaces) by either themselves or else in combination with each other and/or characterized by their relatively high solubility in [the] suitable liquid medium. Paragraph [0002]. US 2004/0105881 specifically discloses that "incorporation of a surfactant into a bilayer membrane that is built from another less soluble amphiphat, such as a phospholipid, can increase the flexibility of the resulting complex membrane ... promot[ing] the capability of complex aggregates ... to cross pores in a semi-permeable membrane that otherwise would prevent comparably large aggregates from crossing." Paragraph [0015]. Barthel et al. : "Randomized controlled trial of disclofenac sodium gel in knee osteoarthritis", Seminars in Arthritis and Rheeumatism, vol. 39, no.3, 1 December 2009, p.203-212, discloses topical treatment of osteoarthritis using diclofenac gel.

**3. SUMMARY OF THE INVENTION**

The present invention provides a vesicular formulation for use in the treatment of pain associated with osteoarthritis comprising one or more phospholipids and one or more non-ionic surfactants and being free of any non-lipid non-surfactant pharmaceutically active agent, wherein the formulation is topically applied.

The vesicular formulation may be for use in the treatment of pain, including deep tissue pain.

The formulation may contain 2.0-10.0% wt phospholipid.

The formulation may contain 0.2-5.0% wt surfactant.

The one or more surfactants may comprise polysorbate 80.

In a further aspect, the present invention provides a vesicular formulation for use in the treatment of pain associated with osteoarthritis which consists essentially of one or more phospholipids and one or more non-ionic surfactants in a pharmaceutically acceptable carrier, wherein the formulation is topically applied.

The molar ratio of phospholipid to surfactant maybe in the range of from about 1:3 to about 30:1, preferably about 1:1 to about 30:1, and more preferably about 2:1 to about 20:1.

The one or more phospholipids may include phosphatidylcholine.

The formulation may comprise one or more non-ionic surfactants selected from poloxyethylene sorbitans, polyhydroxyethylene stearates and polyhydroxyethylene laurylethers.

The formulation may further include one or more buffers, chelators, humectants, lubricants, antioxidants, preservatives, microbiocides, antimicrobials, emollients, co-solvents or thickeners.

Applicants have surprisingly found that vesicular formulations comprising one or more phospholipids and one or more nonionic surfactants are effective in the treatment of pain associated with osteoarthritis, more specifically in the treatment of deep tissue pain, for example osteoarthritis. These vesicular formulations are suitable for topical administration. In some embodiments, these formulations are designed such that the vesicles are able to penetrate deep tissue without diversion into the blood vessels. That is, the formulations are able to travel to the site of the pain in sufficient amount to alleviate that pain to some extent. In accordance with the invention, delivery to the deep tissue includes delivery of the formulation beneath the skin to the muscle tissue and to the joint itself, while limiting systemic delivery and exposure to the formulation.

The formulations of the invention are formulated in the absence of any pharmaceutically active agent, i.e., any non- lipid non-surfactant pharmaceutically active agent that has received regulatory approval for the treatment of pain associated with osteoarthritis. The formulations of the invention do not comprise NSAIDs. The formulations of the invention do not comprise opioids. The formulations of the invention do not comprise COX-2 inhibitors. The formulations of the invention do not comprise any analgesic, for example they do not comprise chlorobutanol, ketamine, oxetacaine, propanidide and thiamylal, aminophenol-derivatives, aminophenazol-derivatives, antranilic acid- and arylpropione acid derivatives, azapropazone, bumadizone, chloroquin- and codeine-derivatives, diclophenac, fentanil, ibuprofen, indometacine, ketoprofen, methadone - substances, morazone, morphine and its derivatives, nifenazone, niflumin acid, pentazozine, pethidine, phenazopyridine, phenylbutazone-derivatives (such as 3,5 pyrazolidine dion), pherazone, piroxicam, propoxyphene, propyphenazon, pyrazol- and phenazone-derivatives (aminophenazone, metamizole, monophenylbutazone, oxyphenebutazone, phenylbutazone or phenazonesalyzilate), salicylic acid-derivatives, sulfasalazine, tilidine; acetylsalicylic acid, ethylmorphine, alclofenac, alphaprodine, aminophenazone, anileridine, azapropazone, benfotiamine, benorilate, benzydamine, cetobemidone, chlorophenesincarbamate, chlorothenoxazine, codeine, dextromoramide, dextro-propoxyphene, ethoheptazine, fentanyl, fenyramidol, fursultiamine, flupirtinmaleate, glafenine, hydromorphone, lactylphenetidine, levorphanol, mefenamic acid, meptazonol, methadone, mofebutazone, nalbufine, Na-salt of noramidopyrinium-methanesulfonate, nefopam, normethadone, oxycodone, paracetamol, pentazocine, pethidine, phenacetine, phenazocine, phenoperidine, pholcodine, piperylone, piritramide, procaine, propyphenazone, salicylamide, thebacone, tiemonium-odide, or tramadone.

As used herein, the term "formulation" is not meant to imply that the ingredients or components are in combination with a pharmaceutically active agent, *i.e.,* any non-lipid non-surfactant active agent that has received regulatory approval for the treatment of pain associated with osteoarthritis.

In one embodiment, the invention provides a vesicular formulation comprising one or more phospholipids and one or more nonionic surfactants and being free of any non-lipid, non-surfactant pharmacuetically active agent for use in the treatment of pain associated with osteoarthritis, more specifically in the treatment of osteoarthritis, optionally in a pharmaceutically acceptable carrier. In one embodiment, the invention provides a vesicular formulation for use in treating pain associated with osteoarthritis of the knee. In one embodiment, the invention provides a formulation that comprises a lysophospholipid, a second phospholipid, such as phosphatidylcholine, and a nonionic surfactant. In one embodiment, the formulation comprises a lysophospholipid in amount from about 4% to about 15% by weight of the total amount of lipid in the formulation. In one embodiment, the invention provides a formulation consisting of one or more phospholipids and one or more nonionic surfactants in a pharmaceutically acceptable carrier. In one embodiment, the invention provides a formulation consisting essentially of one or more phospholipids and one or more nonionic surfactants in a pharmaceutically acceptable carrier, wherein the formulation is topically applied.

Despite the lack of a recognized active agent, the vesicles elicit a therapeutic effect, namely the alleviation or attenuation of pain, for example, on the local deep tissue area. Without being bound by any theory, Applicant believes that the vesicle components themselves are responsible for this affect.

In one embodiment, the invention provides a pharmaceutical package or kit comprising one or more containers filled with the formulation of the invention, and instructions for administration of the formulation to a patient or subject in need thereof for the treatment of pain associated with osteoarthritis, such as deep tissue pain. In certain embodiments, the formulation comprises one or more phospholipids and one or more non-ionic surfactants. In certain embodiments, the formulation comprises a lysophospholipid. The formulation does not comprise a pharmaceutically active agent, *i.e.*, any non-lipid, non-surfactant pharmaceutically active agent that has been approved for the treatment of pain associated with osteoarthritis. In various embodiments, the container comprises a formulation formulated as a suspension, emulsion, gel, cream, lotion, spray, film forming solution or lacquer. The invention provides packages or kits that can be used in any of the above-described methods.

In one embodiment, the invention comprises a vesicular formulation as disclosed herein for use in treating pain assoicated with osteoarthritis, wherein the vesicular formulations of the invention are administered over a period of one or more weeks, for example for at least five weeks, six weeks, seven weeks, eight weeks, nine weeks, ten weeks, eleven weeks, or twelve weeks, sixteen weeks, twenty four weeks, four months, six months, eight months, ten months, one year, two or more years, or indefinitely.

In one embodiment, the formulations of the invention comprise one or more phospholipids, one or more nonionic surfactants, in the absence of any pharmaceutically active agent, *i.e.*, any non-lipid non-surfactant pharmaceutically active agent that has received regulatory approval for the treatment of pain associated with osteoarthritis.

In one embodiment, a 0.1 to 10 gram dose of the formulation of the invention is administered to the patient for the treatment of pain associated with osteoarthritis; a 1 to 10 gram dose of the formulation is administered to the patient for the treatment of pain assoicated with osteoarthritis; a 1 to 5 gram dose of the formulation is administered to the patient for the treatment of pain associated with osteoarthritis; or a 1 gram, 2 gram, 3 gram, 4 gram, 5 gram, 6 gram, 7 gram, 8 gram, 9 gram or 10 gram dose of the formulation is administered to the patient for the treatment of pain associated with osteoarthritis. In some embodiments, the dose is measured as the total weight of the deformasome. In some embodiments, the dose is measured as the total weight of the lipid(s) and non-ionic surfactant(s) in the deformasome. The dose may be administered once or twice daily for the treatment of pain associated with osteoarthritis. The dose may be administered once, twice, three, four, five, six, or seven times per week in accordance with the invention. The dose may be administered every day, every other day, or two to three times a week in accordance with the invention.

The lipid in the pharmaceutical composition is a phospholipid. In some embodiments, the second lipid is a lysophospholipid. The surfactant is a non-ionic surfactant.

In some embodiments, the compositions of the invention form vesicles or other extended surface aggregates (ESAs), wherein the vesicular preparations have improved permeation capability through the semi-permeable barriers, such as skin. The adapatability and deformability of the vesicles allow the vesicles to penetrate beneath the skin to the muscle and the joint itself, however, the size of the vesicle prevents penetration into the vasculature and as a result prevents systemic delivery. While not to be limited to any mechanism of action, the formulations of the invention are able to form vesicles characterized by their deformability and/or adaptability. The adaptability or deformability of the vesicles may be determined by the ability of the vesicles to penetrate a barrier with pores having an average pore diameter at least 50% smaller than the average vesicle diameter before the penetration.

## 4. DETAILED DESCRIPTION OF THE INVENTION

Generally, the nomenclature used herein and the laboratory procedures in organic chemistry, medicinal chemistry, and pharmacology described herein are those well known and commonly employed in the art. Unless defined otherwise, all technical and scientific terms used herein generally have the same meaning as commonly understood by one of ordinary skill in the art to which this disclosure belongs.

The term "subject" refers to an animal, including, but not limited to, a primate (e.g., human), cow, sheep, goat, pig, horse, dog, cat, rabbit, rat, or mouse. The terms "subject" and "patient" are used interchangeably herein in reference, for example, to a mammalian subject, such as a human subject.

As used herein, a "sufficient amount," "amount effective to" or an "amount sufficient to" achieve a particular result refers to an amount of the formulation of the invention is effective to produce a desired effect, which is optionally a therapeutic effect (i.e., by administration of a therapeutically effective amount). Alternatively stated, a "therapeutically effective" amount is an amount that provides some alleviation, mitigation, and/or decrease in at least one clinical symptom. Clinical symptoms associated with the disorder that can be treated by the methods of the disclosure are well-known to those skilled in the art. Further, those skilled in the art will appreciate that the therapeutic effects need not be complete or curative, as long as some benefit is provided to the subject. For example, a "sufficient amount" or "an amount sufficient to" can be an amount that is effective to treat the symptoms of pain associated with osteoarthritis or other joint or muscle pain.

As used herein, the terms "treat", "treating" or "treatment of" mean that the severity of a subject's condition is reduced or at least partially improved or ameliorated and/or that some alleviation, mitigation or decrease in at least one clinical symptom is achieved and/or there is an inhibition or delay in the progression of the condition and/or delay in the progression of the onset of disease or illness. The terms "treat", "treating" or "treatment of" also means managing the disease state.

As used herein, the term "pharmaceutically acceptable" when used in reference to the formulations of the invention denotes that a formulation does not result in an unacceptable level of irritation in the subject to whom the formulation is administered. Preferably such level will be sufficiently low to provide a formulation suitable for approval by regulatory authorities.

As used herein with respect to numerical values, the term "about" means a range surrounding a particular numeral value which includes that which would be expected to result from normal experimental error in making a measurement. For example, in certain embodiments, the term "about" when used in connection with a particular numerical value means $\pm 20\%$, unless specifically stated to be $\pm 1\%$, $\pm 2\%$, $\pm 3\%$, $\pm 4\%$, $\pm 5\%$, $\pm 10\%$, $\pm 15\%$, or $\pm 20\%$ of the numerical value.

The term "alkyl" refers to a linear or branched saturated monovalent hydrocarbon radical, wherein the alkyl may optionally be substituted with one or more substituents Q as described herein. The term "alkyl" also encompasses both linear and branched alkyl, unless otherwise specified. In certain embodiments, the alkyl is a linear saturated monovalent hydrocarbon radical that has 1 to 20 ($C_{1-20}$), 1 to 15 ($C_{1-15}$), 1 to 12 ($C_{1-12}$), 1 to 10 ($C_{1-10}$), or 1 to 6 ($C_{1-6}$) carbon atoms, or a branched saturated monovalent hydrocarbon radical of 3 to 20 ($C_{3-20}$), 3 to 15 ($C_{3-15}$), 3 to 12 ($C_{3-12}$), 3 to 10 ($C_{3-10}$), or 3 to 6 ($C_{3-6}$) carbon atoms. As used herein, linear $C_{1-6}$ and branched $C_{3-6}$ alkyl groups are also referred as "lower alkyl." Examples of alkyl groups include, but are not limited to, methyl, ethyl, propyl (including all isomeric forms), n-propyl, isopropyl, butyl (including all isomeric forms), n-butyl, isobutyl, sec-butyl, t-butyl, pentyl (including all isomeric forms), and hexyl (including all isomeric forms). For example, $C_{1-6}$ alkyl refers to a linear saturated monovalent hydrocarbon radical of 1 to 6 carbon atoms or a branched saturated monovalent hydrocarbon radical of 3 to 6 carbon atoms. It is understood in the chemical arts, that the use of the longer chains described herein may be appropriate, or appropriate only in limited amounts, within a molecule so that the properties of the resulting molecule (such as solubility) are appropriate for the use. Thus, while those in the art may use the above longer length alkyl substituents they will be used only when appropriate to provide the desired function.

The term "aryl" refers to a monocyclic aromatic group and/or multicyclic monovalent aromatic group that contain at least one aromatic hydrocarbon ring. In certain embodiments, the aryl has from 6 to 20 ($C_{6-20}$), from 6 to 15 ($C_{6-15}$), or from 6 to 10 ($C_{6-10}$) ring atoms. Examples of aryl groups include, but are not limited to, phenyl, naphthyl, fluorenyl, azulenyl, anthryl, phenanthryl, pyrenyl, biphenyl, and terphenyl. Aryl also refers to bicyclic or tricyclic carbon rings, where one of the rings is aromatic and the others of which may be saturated, partially unsaturated, or aromatic, for example, dihydronaphthyl, indenyl, indanyl, or tetrahydronaphthyl (tetralinyl). In certain embodiments, aryl may also be optionally substituted with one or more substituents Q as described herein.

The term "heteroaryl" refers to a monocyclic aromatic group and/or multicyclic aromatic group that contain at least one aromatic ring, wherein at least one aromatic ring contains one or more heteroatoms independently selected from O, S, and N. Each ring of a heteroaryl group can contain one or two O atoms, one or two S atoms, and/or one to four N atoms, provided that the total number of heteroatoms in each ring is four or less and each ring contains at least one carbon atom. The heteroaryl may be attached to the main structure at any heteroatom or carbon atom which results in the creation of a stable compound. In certain embodiments, the heteroaryl has from 5 to 20, from 5 to 15, or from 5 to 10 ring atoms. Examples of monocyclic heteroaryl groups include, but are not limited to, pyrrolyl, pyrazolyl, pyrazolinyl, imidazolyl, oxazolyl, isoxazolyl, thiazolyl, thiadiazolyl, isothiazolyl, furanyl, thienyl, oxadiazolyl, pyridyl, pyrazinyl, pyrimidinyl, pyridazinyl, and triazinyl. Examples of bicyclic heteroaryl groups include, but are not limited to, indolyl, benzothiazolyl, benzoxazolyl, benzothienyl, quinolinyl, tetrahydroisoquinolinyl, isoquinolinyl, benzimidazolyl, benzopyranyl, indolizinyl, benzofuranyl, isobenzofuranyl, chromonyl, coumarinyl, cinnolinyl, quinoxalinyl, indazolyl, purinyl, pyrrolopyridinyl, furopyridinyl, thienopyridinyl, dihydroisoindolyl, and tetrahydroquinolinyl. Examples of tricyclic heteroaryl groups include, but are not limited to, carbazolyl, benzindolyl, phenanthrollinyl, acridinyl, phenanthridinyl, and xanthenyl. In certain embodiments, heteroaryl may also be optionally substituted with one or more substituents Z as described herein.

The term "alkenoyl" as used herein refers to -C(O)-alkenyl. The term "alkenyl" refers to a linear or branched monovalent hydrocarbon radical, which contains one or more, in one embodiment, one to five, carbon-carbon double bonds. The alkenyl may be optionally substituted with one or more substituents Z as described herein. The term "alkenyl" also embraces radicals having "*cis*" and "*trans*" configurations, or alternatively, "Z" and "E" configurations, as appreciated by those of ordinary skill in the art. As used herein, the term "alkenyl" encompasses both linear and branched alkenyl, unless otherwise specified. For example, $C_{2-6}$ alkenyl refers to a linear unsaturated monovalent hydrocarbon radical of 2 to 6 carbon atoms or a branched unsaturated monovalent hydrocarbon radical of 3 to 6 carbon atoms. In certain embodiments, the alkenyl is a linear monovalent hydrocarbon radical of 2 to 30 ($C_{2-30}$), 2 to 24 ($C_{2-24}$), 2 to 20 ($C_{2-20}$), 2 to 15 ($C_{2-15}$), 2 to 12 ($C_{2-12}$), 2 to 10 ($C_{2-10}$), or 2 to 6 ($C_{2-6}$) carbon atoms, or a branched monovalent hydrocarbon radical of 3 to 30 ($C_{3-30}$), 3 to 24 ($C_{3-24}$), 3 to 20 ($C_{3-20}$), 3 to 15 ($C_{3-15}$), 3 to 12 ($C_{3-12}$), 3 to 10 ($C_{3-10}$), or 3 to 6 ($C_{3-6}$) carbon atoms. Examples of alkenyl groups include, but are not limited to, ethenyl, propen-1-yl, propen-2-yl, allyl, butenyl, and 4-methylbutenyl. In certain embodiments, the alkenoyl is mono-alkenoyl, which contains one carbon-carbon double bond. In certain embodiments, the alkenoyl is di-alkenoyl, which contains two carbon-carbon double bonds. In certain embodiments, the alkenoyl is poly-alkenoyl, which contains more than two carbon-carbon double bonds.

The term "heterocyclyl" or "heterocyclic" refers to a monocyclic non-aromatic ring system and/or multicyclic ring system that contains at least one non-aromatic ring, wherein one or more of the non-aromatic ring atoms are heteroatoms independently selected from O, S, or N; and the remaining ring atoms are carbon atoms. In certain embodiments, the heterocyclyl or heterocyclic group has from 3 to 20, from 3 to 15, from 3 to 10, from 3 to 8, from 4 to 7, or from 5 to 6 ring atoms. In certain embodiments, the heterocyclyl is a monocyclic, bicyclic, tricyclic, or tetracyclic ring system, which may include a fused or bridged ring system, and in which the nitrogen or sulfur atoms may be optionally oxidized, the nitrogen atoms may be optionally quaternized, and some rings may be partially or fully saturated, or aromatic. The heterocyclyl may be attached to the main structure at any heteroatom or carbon atom which results in the creation of a stable compound. Examples of such heterocyclic radicals include, but are not limited to, acridinyl, azepinyl, benzimidazolyl, benzindolyl, benzoisoxazolyl, benzisoxazinyl, benzodioxanyl, benzodioxolyl, benzofuranonyl, benzofuranyl, benzonaphthofuranyl, benzopyranonyl, benzopyranyl, benzotetrahydrofuranyl, benzotetrahydrothienyl, benzothiadiazolyl, benzothiazolyl, benzothiophenyl, benzotriazolyl, benzothiopyranyl, benzoxazinyl, benzoxazolyl, benzothiazolyl, β-carbolinyl, carbazolyl, chromanyl, chromonyl, cinnolinyl, coumarinyl, decahydroisoquinolinyl, dibenzofuranyl, dihydrobenzisothiazinyl, dihydrobenzisoxazinyl, dihydrofuryl, dihydropyranyl, dioxolanyl, dihydropyrazinyl, dihydropyridinyl, dihydropyrazolyl, dihydropyrimidinyl, dihydropyrrolyl, dioxolanyl, 1,4-dithianyl, furanonyl, furanyl, imidazolidinyl, imidazolinyl, imidazolyl, imidazopyridinyl, imidazothiazolyl, indazolyl, indolinyl, indolizinyl, indolyl, isobenzotetrahydrofuranyl, isobenzotetrahydrothienyl, isobenzothienyl, isochromanyl, isocoumarinyl, isoindolinyl, isoindolyl, isoquinolinyl, isothiazolidinyl, isothiazolyl, isoxazolidinyl, isoxazolyl, morpholinyl, naphthyridinyl, octahydroindolyl, octahydroisoindolyl, oxadiazolyl, oxazolidinonyl, oxazolidinyl, oxazolopyridinyl, oxazolyl, oxiranyl, perimidinyl, phenanthridinyl, phenathrolinyl, phenarsazinyl, phenazinyl, phenothiazinyl, phenoxazinyl, phthalazinyl, piperazinyl, piperidinyl, 4-piperidonyl, pteridinyl, purinyl, pyrazinyl, pyrazolidinyl, pyrazolyl, pyridazinyl, pyridinyl, pyridopyridinyl, pyrimidinyl, pyrrolidinyl, pyrrolinyl, pyrrolyl, quinazolinyl, quinolinyl, quinoxalinyl, quinuclidinyl, tetrahydrofuryl, tetrahydrofuranyl, tetrahydroisoquinolinyl, tetrahydropyranyl, tetrahydrothienyl, tetrazolyl, thiadiazolopyrimidinyl, thiadiazolyl, thiamorpholinyl, thiazolidinyl, thiazolyl, thienyl, triazinyl, triazolyl, and 1,3,5-trithianyl. In certain embodiments, heterocyclic may also be optionally substituted with one or more substituents Z as described herein.

The term "halogen", "halide" or "halo" refers to fluorine, chlorine, bromine, and/or iodine.

The term "optionally substituted" is intended to mean that a group, including alkyl, alkenyl, alkynyl, cycloalkyl, aryl, aralkyl, heteroaryl, and heterocyclyl, may be substituted with one or more substituents Z, in one embodiment, one, two, three or four substituents Z, where each Z is independently selected from the group consisting of cyano, halo, oxo, nitro, $C_{1-6}$ alkyl, halo-$C_{1-6}$ alkyl, $C_{2-6}$ alkenyl, $C_{2-6}$ alkynyl, $C_{3-7}$ cycloalkyl, $C_{6-14}$ aryl, $C_{7-14}$ aralkyl, heteroaryl, heterocyclyl, -C(O)$R^e$, -C(O)O$R^e$, -C(O)N$R^fR^g$, - C(N$R^e$)N$R^fR^g$, -O$R^e$, - OC(O)$R^e$, -OC(O)O$R^e$, -OC(O)N$R^fR^g$, -OC(=N$R^e$)N$R^fR^g$, -OS(O)$R^e$, -OS(O)$_2R^e$, - OS(O)N$R^fR^g$, -OS(O)$_2$N$R^fR^g$, -N$R^fR^g$, -N$R^e$C(O)$R^f$, -N$R^e$C(O)O$R^f$, -N$R^e$C(O)N$R^fR^g$, -N$R^e$C(=N$R^h$)N$R^fR^g$, -N$R^e$S(O)$R^f$, -N$R^e$S(O)$_2R^f$, -N$R^e$S(O)N$R^fR^g$, - N$R^e$S(O)$_2$N$R^fR^g$, -S$R^e$, -S(O)$R^e$, -S(O)$_2R^e$, and -S(O)$_2$N$R^fR^g$, wherein each $R^e$, $R^f$, $R^g$, and $R^h$ is independently hydrogen, $C_{1-6}$ alkyl, $C_{2-6}$ alkenyl, $C_{2-6}$ alkynyl, $C_{3-7}$ cycloalkyl, $C_{6-14}$ aryl, $C_{7-14}$ aralkyl, heteroaryl, or heterocyclyl; or $R^f$ and $R^g$ together with the N atom to which they are attached form heterocyclyl.

The term "solvate" refers to a compound provided herein or a salt thereof, which further includes a stoichiometric or non-stoichiometric amount of solvent bound by noncovalent intermolecular forces. Where the solvent is water, the solvate is a hydrate.

In accordance with this disclosure, the term "comprising" is inclusive or openended and does not exclude additional, unrecited elements or method steps; the term "consiting of" excludes any element, step, or ingredient not specified; and the term "consisting essentially of" excludes any element, step, or ingredient that materially changes a basic characteristic of the invention.

The formulation of the invention provided herein comprises at least one phospholipid, at least one nonionic surfactant, optionally suspended in a pharmaceutically acceptable medium, preferably an aqueous solution, preferably having a pH ranging from 3.5 to 9.0, preferably from 4 to 7.5. The formulation of the invention may optionally contain buffers, antioxidants, preservatives, microbicides, antimicrobials, emollients, co-solvents, and/or thickeners. In some embodiments, the formulation of the invention comprises a mixture of more than one phospholipid. In some embodiments, the formulation of the invention consists essentially of at least one phospholipid, at least one nonionic surfactant, a pharmaceutically acceptable carrier, and optionally buffers, antioxidants, preservatives, microbicides, antimicrobials, emollients, co-solvents, and/or thickeners. In some embodiments, the formulation of the invention consists of at least one phospholipid, at least one nonionic surfactant, a pharmaceutically acceptable carrier, and one or more of the following: buffers, antioxidants, preservatives, microbicides, antimicrobials, emollients, co-solvents, and thickeners.

## 4.1. LIPID

The lipid used in the formulation according to the present invention is a phospholipid.

In the sense of this disclosure, a "lipid" is any substance, which has properties like or similar to those of a fat. As a rule, it has an extended apolar group (the "chain", X) and generally also a water-soluble, polar hydrophilic part, the "head" group (Y) and has the basic Formula I:

$$X\text{-}Y_n \qquad \textbf{(I)}$$

wherein n is equal to or larger than zero.

Lipids with n=0 are referred to as apolar lipids and lipids with n>1 are referred to as polar lipids. In this sense, all amphiphilic substances, including, but not limited to glycerides, glycerophospholipids, glycerophosphinolipids, glycerophosphonolipids, sulfolipids, sphingolipids, isoprenoid lipids, steroids or sterols and carbohydrate-containing lipids can generally be referred to as lipids. A list of relevant lipids and lipid related definitions is provided in EP 0 475 160 A1 (*see, e.g.* p. 4, 1. 8 to p. 6, 1. 3) and U.S. Patent No. 6,165,500 (*see, e.g.,* col. 6, 1. 10 to col. 7, 1. 58).

A phospholipid is, for example, a compound of Formula II:

**EP 2 437 726 B1**

$$R^1\text{-}CH_2\text{-}CHR^2\text{-}CR^3H\text{-}O\text{-}PHO_2\text{-}O\text{-}R^4 \qquad \textbf{(II)}$$

wherein $R^1$ and $R^2$ cannot both be hydrogen, OH or a $C_1$-$C_3$ alkyl group, and typically are independently, an aliphatic chain, most often derived from a fatty acid or a fatty alcohol; $R^3$ generally is a hydrogen.

The OH-group of the phosphate is a hydroxyl radical or hydroxyl anion (*i.e.*, hydroxide) form, dependent on degree of the group ionization. Furthermore, $R^4$ may be a proton or a short-chain alkyl group, substituted by a tri-short-chain alkylammonium group, such as a trimethylammonium group, or an amino-substituted short-chain alkyl group, such as 2-trimethylammonium ethyl group (cholinyl) or 2-dimethylammonium short alkyl group.

A sphingophospholipid is, for example, a compound of Formula IIB:

$$R^1\text{-}Sphingosine\text{-}O\text{-}PHO_2\text{-}O\text{-}R^4 \qquad \textbf{(IIB)}$$

wherein $R^1$ is a fatty-acid attached via an amide bond to the nitrogen of the sphingosine and $R^4$ has the meanings given under Formula II.

A lipid preferably is a substance of formulaeII or IIB, wherein $R^1$ and/or $R^2$ are acyl or alkyl, n-hydroxyacyl or n-hydroxyalkyl, but may also be branched, with one or more methyl groups attached at almost any point of the chain; usually, the methyl group is near the end of the chain (iso or anteiso). The radicals $R^1$ and $R^2$ may moreover either be saturated or unsaturated (mono-, di- or poly-unsaturated). $R^3$ is hydrogen and $R^4$ is 2-trimethylammonium ethyl (the latter corresponds to the phosphatidyl choline head group), 2-dimethylammonium ethyl, 2-methylammonium ethyl or 2-aminoethyl (corresponding to the phosphatidyl ethanolamine head group). $R^4$ may also be a proton (giving phosphatidic acid), a serine (giving phosphatidylserine), a glycerol (giving phosphatidylglycerol), an inositol (giving phosphatidylinositol), or an alkylamine group (giving phosphatidylethanolamine in case of an ethylamine), if one chooses to use a naturally occurring glycerophospholipid. Otherwise, any other sufficiently polar phosphate ester, such that will form a lipid bilayer, may be considered as well for making the formulations of the disclosure.

Table 1 lists preferred phospholipids in accordance with one embodiment of the disclosure.

Table 1

## Preferred (phospho)lipids

| Fatty chain | | Phospholipid: Type and Charge | | | | | |
|---|---|---|---|---|---|---|---|
| Name(s) | Length: nr. of double bonds | Phosphatidylcholine / ± | Phosphatidylethanolamine / ± | Sphingomyelin / + | Phosphatidylglycerol / - | Phosphatidylinositol / - | Phosphatidic acid / - |
| | | Main lipid, L1 | Main lipid, L1 | Main lipid, L1 | Aux. lipid, L2 | Aux. lipid, L2 | Aux. lipid, L2 |
| | C24 | | | | | | |
| Behen(o)yl | C22 | | | | | | |
| Eruca(o)yl | C22:1-13cis | | | | | | |
| Arachin(o)yl | C20 | | | | | | |
| Gadolen(o)yl | C20:1-11cis | | | | | | |
| Arachidon(o)yl | C20:4-5,8,11,14cis | | | | | | |
| Ole(o)yl | C18:1-9cis | DOPC | DOPE | SM-oleyl | DOPG | DOPI | DOPA |
| Stear(o)yl | C18 | | | | | | |
| Linol(o)yl | C18:2-9,12cis | (Soy-PC / | (Soy-PE / | Brain SM | (Soy-PC / | (Soy-PI / | (Soy-PA / |
| Linole(n/o)yl | C18:3-9,12,15cis | Egg-PC) | Egg-PE) | | Egg-PC) | Liver-PI) | Egg-PA) |
| Palmitole(o)yl | C18:1-9cis | | | | | | |
| Palmit(o)yl | C16 | | | | | | |
| Myrist(o)yl | C14 | DMPC | DMPE | SM-myristyl | DMPG | DMPI | |
| Laur(o)yl | C12 | DLPC | DLPE | SM-lauryl | | | DLPA |
| Capr(o)yl | C10 | | | | | | |
| Rel. concentration range L1/L2 (M/M) | | 1/0 | 1/0 | 10/1-1/1 | 10/1-3/1 | 10/1-5/1 | |
| "Total Lipid"* concentration range (w-%) | | 0.5-45 | 0.5-45 | 0.5-40 | 0.5-40 | 0.5-40 | |

*Total Lipid includes phospholipid(s), surfactant(s)t and all lipophilic excipients

The preferred lipids in the context of this disclosure are uncharged and form stable, well hydrated bilayers; phosphatidylcholines, phosphatidylethanolamine, and sphingomyelins are the most prominent representatives of such lipids. Any of those can have chains as listed in the Table 1, the ones forming fluid phase bilayers, in which lipid chains are in disordered state, being preferred.

Different negatively charged, *i.e.*, anionic, lipids can also be incorporated into vesicular lipid bilayers. Attractive examples of such charged lipids are phosphatidylglycerols, phosphatidylinositols and, somewhat less preferred, phosphatidic acid (and its alkyl ester) or phosphatidylserine. It will be realized by anyone skilled in the art that it is less commendable to make vesicles just from the charged lipids than to use them in a combination with electro- neutral bilayer component(s). In case of using charged lipids, buffer composition and/or pH care must selected so as to ensure the desired degree of lipid head-group ionization and/or the desired degree of electrostatic interaction between the, oppositely, charged drug and lipid molecules. Moreover, as with neutral lipids, the charged bilayer lipid components can in principle have any of the chains listed in the Table 1. The chains forming fluid phase lipid bilayers are clearly preferred, however, both due to vesicle adaptability increasing role of increasing fatty chain fluidity and due to better ability of lipids in fluid phase to mix with each other.

The fatty acid- or fatty alcohol-derived chain of a lipid is typically selected amongst the basic aliphatic chain types given in the following tables:

Table 2: The (most) preferred basic, straight, saturated fatty chain residues

| Shorthand designation | Systematic name | Trivial name |
|---|---|---|
| 12:0 | Dodecanoic | Laurie |
| 13:0 | Tridecanoic | |
| 14:0 | Tetradecanoic | Myristic |
| 15:0 | Pentadecanoic | |
| 16:0 | Hexadecanoic | Palmitic |
| 17:0 | Heptadecanoic | Margaric |
| 18:0 | Octadecanoic | Stearic |
| 19:0 | Nonadecanoic | |
| 20:0 | Eicosanoic | Arachidic |
| 21:0 | Heneicosanoic | |
| 22:0 | Docosanoic | Behenic |
| 23:0 | Tricosanoic | |
| 24:0 | Tetracosanoic | Lignoceric |

Table 3: The (most) preferred monoenoic fatty chain residues

| Shorthand designation | Systematic name | Trivial name |
|---|---|---|
| 9-14:1 / 14:1(n-5) | cis-9-Tetradecenoic | Myristoleic |
| 7-16:1 / 16:1(n-9) | cis-7-Hexadecenoic | |
| 9-16:1 / 16:1(n-7) | cis-9-Hexadecenoic | Palmitoleic |
| 9-18:1 / 18:1(n-9) | cis-9-Octadecenoic | Oleic |
| 11-18:1 / 18:1(n-7) | cis-11-Octadecenoic | cis-Vaccenic |
| 11-20:1 / 20:1(n-9) | cis-11-Eicosenoic | Gondoic |
| 14-20:1 / 20:1(n-6) | cis-14-Eicosaenoic | |
| 13-22:1 / 22:1(n-9) | cis-13-Docosenoic | Erucic |
| 15-24:1 / 24:1(n-9) | cis-15-Tetracosenoic | Nervoni |
| 3t-18:1 | trans-3-Hexadecenoi | |
| 9t-18:1 | trans-9-Octadecenoic | Elaidic |
| 11t-18:1 | trans-11-Octadecenoic | Vaccenic |

Table 4: The (most) preferred dienoic and polyenoic fatty chain residues

| Shorthand designation | Systematic name | Trivial name |
|---|---|---|
| 10,13c-16:2 / 16:2(n-3) | 10-cis,13-cis-Hexadecadienoic | |

(continued)

| Shorthand designation | Systematic name | Trivial name |
|---|---|---|
| 7,10c-16:2 / 16:3(n-6) | 7-cis,10-cis-Hexadecadienoic | |
| 7,10,13c-16:3 / 16:3(n-3) | 7-cis,10-cis,13-cis-Hexadecatrienoic | |
| 12,15c-18:2 / 18:2(n-3) | 12-cis,15-cis-Octadecadienoic | $\alpha$-Linoleic |
| 10,12t-18:2 / 18:2(n-6) | trans-10,trans-12-Octadecadienoic | |
| 9,12c-18:2 / 18:2(n-6) | 9-cis,12-cis-Octadecadienoic | $\gamma$-Linoleic |
| 9,12,15c-18:3 / 18:3(n-3) | 9-cis,12-cis,15-cis-Octadecatrienoic | $\alpha$-Linolenic |
| 6,9,12c-19:3 / 18:3(n-6) | 6-cis,9-cis,12-cis-Octadecatrienoic | $\gamma$-Linolenic |
| 9c,11c,13t-18:3 | 9-cis-,11-trans,13-trans-Octadecatrienoic | $\gamma$-Eleostearic |
| 8t,10t,12c-18:3 | 8-trans,10-trans,12-cis-Octadecatrienoic | Calendic |
| 6,9,12,15c-18:4 / 18:4(n-3) | 6,9,12,15-Octadecatetraenoic | Stearidonic |
| 3,6,9,12c-18:4 / 18:4(n-6) | 3,6,9,12-Octadecatetraenoic | |
| 3,6,9,12,15c-18:5 / 18:5(n-3) | 3,6,9,12,15-Octadecapentaenoic | |
| 14,17c-20:2 / 20:2(n-3) | 14-cis,17-cis-Eicosadienoic | |
| 11,14c-20:2 / 20:2(n-6) | 11-cis,14-cis-Eicosadienoic | |
| 11,14,17r-2D:3 / 20:3(n-3) | 8-cis,11-cis,14-cis-Eicosatrienoic | Dihomo-$\alpha$-linolenic |
| 8,11,14c-20:3 / 20:3(n-6) | 8-cis,11-cis,14-cis-Eicosatrienoic | Dihomo-$\gamma$-linolenic |
| 5,8,11c-20:3 20:3(n-9) | 5,8,11all-cis-Eicosatrienoic | 'Mead's' |
| 5,8,11,14c-20:4 / 20:4(n-6) | 5,8,11;14-all-cis-Eicosatetraenoic | Arachidonic |
| 8,11,14,17c-20:4 / 20:4(n-3) | 8,11,14,17-all-cis-Eicosatetraenoic | |
| 5,8,11,14417c-20:5 or 20:5(n-3) | 5,8,11,14,17-all-cis-Eicosapentaenoic | |
| 13,16c-22:2 | 13,16-Docosadienoic | |
| 13,16,19c-22:3 / 22:3(n-3) | 13,16,19-Docosatrienoic | |
| 10,13,16c-22:3 / 22:3(n-6) | 10,13,16-Docosatrienoic | |
| 7,10,t3,16c-22:4 / 22:4(n-6) | 7,10,13,16-Docosatetraenoic | Adrenic |
| 4,7,10,13,16e-22:5 or 22:5(n-6) | 4,7,10,13,16-Docosapentaenoic | |
| 4,7,10,13,16,19c-22:5 or 22:5(n-3) | 4,7,10,13,16,19-Docosahexaenoic | |

Other double bond combinations or positions are possible as well.

Suitable fatty residues can furthermore be branched, for example, can contain a methyl group in an iso or anteiso position of the fatty acid chain, or else closer to the chain middle, as in 10-$R$-methyloctadecanoic acid or tuberculostearic chain. Relatively important amongst branched fatty acids are also isoprenoids, many of which are derived from 3,7,1 1,15-tetramethylhexadec-trans-2-en-1-ol, the aliphatic alcohol moiety of chlorophyll. Examples include 5,9,13,17-tetramethyloctadecanoic acid and especially 3,7,11,15- tetramethylhexadecanoic (phytanic) and 2,6,10,14-tetramethylpentadecanoic (pristanic) acids. A good source of 4,8,12-trimethyltridecanoic acid are marine organisms. Combination of double bonds and side chains on a fatty residue are also possible.

Alternatively, suitable fatty residues may carry one or a few oxy- or cyclic groups, especially in the middle or towards the end of a chain. The most prominent amongst the later, alicyclic fatty acids, are those comprising a cyclopropane (and sometimes cyclopropene) ring, but cyclohexyl and cycloheptyl rings can also be found and might be useful for purposes of this disclosure. 2-(D)-Hydroxy fatty acids are more ubiquitous than alicyclic fatty acids, and are also important constituents of sphingolipids. Also interesting are 15-hydroxyhexadecanoic and 17-hydroxy-octadecanoic acids, and maybe 9-hydroxy-octadeca-$trans$10,$trans$-12-dienoic (dimorphecolic) and 13-hydroxy-octadeca-$cis$-9,$trans$-11-dienoic (coriolic) acid. Arguably the most prominent hydroxyl-fatty acid in current pharmaceutical use is ricinoleic acid, (D-(-)12-hydroxy-octadec-$cis$-9-enoic acid, which comprises up to 90% of castor oil, which is also often used in hydrogenated form. Epoxy-, methoxy-, and furanoid-fatty acids are of only limited practical interest in the context of this disclosure.

Generally speaking, unsaturation, branching or any other kind of derivatization of a fatty acid is best compatible with the intention of present disclosure of the site of such modification is in the middle or terminal part of a fatty acid chain. The $cis$-unsaturated fatty acids are also more preferable than $trans$-unsaturated fatty acids and the fatty radicals with fewer double bonds are preferred over those with multiple double bonds, due to oxidation sensitivity of the latter. Moreover, symmetric chain lipids are generally better suited than asymmetric chain lipids.

A preferred lipid of the Formula II is, for example, a natural phosphatidylcholine, which used to be called lecithin. It can be obtained from egg (rich in palmitic, $C_{16:0}$, and oleic, $C_{18:1}$, but also comprising stearic, $C_{18:0}$, palmitoleic, $C_{16:1}$,

linolenic, $C_{18:2}$, and arachidonic, $C_{20:4}$, radicals), soybean (rich in unsaturated $C_{18}$ chains, but also containing some palmitic radical, amongst a few others), coconut (rich in saturated chains), olives (rich in monounsaturated chains), saffron (safflower) and sunflowers (rich in n-6 linoleic acid), linseed (rich in n-3 linolenic acid), from whale fat (rich in monounsaturated n-3 chains), from primrose or primula (rich in n-3 chains). Preferred, natural phosphatidyl ethanolamines (used to be called cephalins) frequently originate from egg or soybeans. Preferred sphingomyelins of biological origin are typically prepared from eggs or brain tissue. Preferred phosphatidylserines also typically originate from brain material whereas phosphatidylglycerol is preferentially extracted from bacteria, such as E. Coli, or else prepared by way of transphosphatidylation, using phospholipase D, starting with a natural phosphatidylcholine. The preferably used phosphatidylinositols are isolated from commercial soybean phospholipids or bovine liver extracts. The preferred phosphatidic acid is either extracted from any of the mentioned sources or prepared using phospholipase D from a suitable phosphatidylcholine.

Furthermore, synthetic phosphatidyl cholines ($R^4$ in Formula II corresponds to 2-trimethylammonium ethyl), and $R^1$ and $R^2$ are aliphatic chains, as defined in the preceding paragraph with 12 to 30 carbon atoms, preferentially with 14 to 22 carbon atoms, and even more preferred with 16 to 20 carbon atoms, under the proviso that the chains must be chosen so as to ensure that the resulting ESAs comprise fluid lipid bilayers. This typically means use of relatively short saturated and of relatively longer unsaturated chains. Synthetic sphingomyelins ($R^4$ in Formula IIB corresponds to 2-trimethylammonium ethyl), and $R^1$ is an aliphatic chain, as defined in the preceding paragraph, with 10 to 20 carbon atoms, preferentially with 10 to 14 carbon atoms per fully saturated chain and with 16-20 carbon atoms per unsaturated chain.

Synthetic phosphatidyl ethanolamines ($R^4$ is 2-aminoethyl), synthetic phosphatidicacids ($R^4$ is a proton) or its ester ($R^4$ corresponds, for example, to a short-chain alkyl, such as methyl or ethyl), synthetic phosphatidyl serines ($R^4$ is L- or D-serine), or synthetic phosphatidyl (poly)alcohols, such as phosphatidyl inositol, phosphatidyl glycerol ($R^4$ is L- or D-glycerol) are preferred as lipids, wherein $R^1$ and $R^2$ are fatty residues of identical or moderately different type and length, especially such as given in the corresponding tables given before in the text. Moreover, $R^1$ can represent alkenyl and $R^2$ identical hydroxyalkyl groups, such as tetradecylhydroxy or hexadecylhydroxy, for example, in ditetradecyl or dihexadecylphosphatidyl choline or ethanolamine, $R^1$ can represent alkenyl and $R^2$ hydroxyacyl, such as a plasmalogen ($R^4$ trimethylammonium ethyl), or $R^1$ can be acyl, such as lauryl, myristoyl or palmitoyl and $R^2$ can represent hydroxy as, for example, in natural or synthetic lysophosphatidyl cholines or lysophosphatidyl glycerols or lysophosphatidyl ethanolamines, such as 1-myristoyl or 1-palmitoyllysophosphatidyl choline or -phosphatidyl ethanolamine; frequently, $R^3$ represents hydrogen.

A lipid of Formula IIB is also a suitable lipid within the sense of this disclosure.
In Formula IIB, n=1, $R^1$ is an alkenyl group, $R^2$ is an acylamido group, $R^3$ is hydrogen and $R^4$ represents 2-trimethyl-ammonium ethyl (choline group). Such a lipid is known under the name of sphingomyelin.

Suitable lipids furthermore are a lysophosphatidyl choline analog, such as 1-lauroyl-1,3-dihydroxypropane-3-phosphoryl choline, a monoglyceride, such as monoolein or monomyristin, a cerebroside, ceramide polyhexoside, sulfatide, sphingoplasmalogen, a ganglioside or a glyceride, which does not contain a free or esterified phosphoryl or phosphono or phosphino group in the 3 position. An example of such a glyceride is diacylglyceride or 1-alkenyl-1-hydroxy-2-acyl glyceride with any acyl or alkenyl groups, wherein the 3-hydroxy group is etherified by one of the carbohydrate groups named, for example, by a galactosyl group such as a monogalactosyl glycerin.

Lipids with desirable head or chain group properties can also be formed by biochemical means, for example, by means of phospho lipases (such as phospholilpase A1, A2, B, C and, in particular, D), desaturases, elongases, acyl transferases, etc., from natural or synthetic precursors.

Furthermore, a suitable lipid is any lipid, which is contained in biological membranes and can be extracted with the help of apolar organic solvents, such as chloroform. Aside from the lipids already mentioned, such lipids also include, for example, steroids, such as estradiol, or sterols, such as cholesterol, beta-sitosterol, desmosterol, 7-keto-cholesterol or beta-cholestanol, fat-soluble vitamins, such as retinoids, vitamins, such as vitamin A1 or A2, vitamin E, vitamin K, such as vitamin K1 or K2 or vitamin D1 or D3, etc.

The less soluble amphiphilic components comprise or preferably comprise a synthetic lipid, such as myristoleoyl, palmitoleoyl, petroselinyl, petroselaidyl, oleoyl, elaidyl, cis- or trans-vaccenoyl, linolyl, linolenyl, linolaidyl, octadecatetraenoyl, gondoyl, eicosaenoyl, eicosadienoyl, eicosatrienoyl, arachidoyl, cis- or trans-docosaenoyl, docosadienoyl, docosatrienoyl, docosatetraenoyl, lauroyl, tridecanoyl, myristoyl, pentadecanoyl, palmitoyl, heptadecanoyl, stearoyl or nonadecanoyl, glycerophospholipid or corresponding derivatives with branched chains or a corresponding dialkyl or sphingosin derivative, glycolipid or other diacyl or dialkyl lipid.

The more soluble amphiphilic components(s) is/are frequently derived from the less soluble components listed above and, to increase the solubility, substituted and/or complexed and/or associated with a butanoyl, pentanoyl, hexanoyl, heptanoyl, octanoyl, nonanoyl, decanoyl or undecanoyl substituent or several, mutually independent, selected substituents or with a different material for improving the solubility.

A further suitable lipid is a diacyl- or dialkyl-glycerophosphoetha- nolamine azo polyethoxylene derivative, a didecanoylphosphatidyl choline or a diacylphosphoolligomaltobionamide.

In certain embodiments, the amount of lipid in the formulation is from about 1% to about 12%, about 1% to about 10%, about 1% to about 4%, about 4% to about 7% or about 7% to about 10% by weight. The lipid is a phospholipid. In another specific embodiment, the phospholipid is a phosphatidylcholine.

In some embodiments, the lipid in the formulation does not comprise an alkyl-lysophospholipid. In some embodiments, the lipid in the formulation does not comprise a polyeneylphosphatidylcholine.

## 4.2. SURFACTANT

The surfactants used in the formulation according to the present invention are non-ionic surfactants.

The term "surfactant" has its usual meaning. A list of relevant surfactants and surfactant related definitions is provided in EP 0 475 160 A1 (*see, e.g.,* p. 6, 1. 5 to p.14. 1.17) and U.S. Pat. No. 6,165,500 (*see, e.g.*, col. 7, 1. 60 to col. 19, 1. 64), and in appropriate surfactant or pharmaceutical Handbooks, such as Handbook of Industrial Surfactants or US Pharmacopoeia, Pharm. Eu. In some embodiments, the surfactants are those described in Tables 1-18 of U.S. Patent Application Publication No. 2002/0012680 A1, published January 31, 2002. The following list therefore only offers a selection, which is by no means complete or exclusive, of several non-ionic surfactant classes that are particularly common or useful in conjunction with present patent application. Preferred surfactants to be used in accordance with the disclosure include those with an HLB greater than 12. The list includes polyethylen-glycol-acylphenyl ethers, especially nonaethylen-glycol-octyl- phenyl ether, polyethylene-long fatty chain-ethers, especially polyethylene-acyl ethers, such as nonaethylendecyl ether, nonaethylen-dodecyl ether or octaethylene-dodecyl ether, polyethyleneglycol-isoacyl ethers, such as octaethyleneglycol-isotridecyl ether, polyethyleneglycol-sorbitane-long fatty chain esters, for example polyethyleneglycol-sorbitane-acyl esters and especially polyoxyethylene-monolaurate (e.g. polysorbate 20 or Tween 20), polyoxyethylene-sorbitan-monooleate (e.g. polysorbate 80 or Tween 80), polyoxyethylene-sorbitan-monolauroleylate, polyoxyethylene - sorbitan-monopetroselinate, polyoxyethylene -sorbitan- monoelaidate, polyoxyethylene - sorbitan-myristoleylate, polyoxyethylene -sorbitan-palmitoleinylate, polyoxyethylene- sorbitan-p-etroselinylate, polyhydroxyethylene-long fatty chain ethers, for example polyhydroxyethylene-acyl ethers, such as polyhydroxyethylene-lauryl ethers, polyhydroxyethylene-myristoyl ethers, polyhydroxyethylene-cetylst-earyl, polyhyd roxyethylene-palmityl ethers, polyhyd roxyethyleneoleoyl ethers, polyhydroxyethylene- palmitoleoyl ethers, polyhydroxyethylene-lino- leyl, polyhydroxyethylen-4, or 6, or 8, or 10, or 12-lauryl, miristoyl, palmitoyl, palmitoleyl, oleoyl or linoeyl ethers (Brij series), or in the corresponding esters, polyhydroxyethylen-laurate, -myristate, -palmitate, -stearate or -oleate, especially polyhydroxyethylen-8-stearate (Myrj 45) and polyhydroxyethylen-8-oleate, polyethoxylated castor oil 40 (Cremophor EL), sorbitane-mono long fatty chain, for example alkylate (Arlacel or Span series), especially as sorbitane-monolaurate (Arlacel 20, Span 20), long fatty chain, for example acyl-N-methylglucamides, alkanoyl-N-methylglucamides, especially decanoyl-N-methylglucamide, dodecanoyl-N-methylglucamide, long fatty chain thioglucosides, such as alkylthioglucosides and especially heptyl-, octyl- and nonyl-beta-D-thioglucopyranoside; long fatty chain derivatives of various carbohydrates, such as pentoses, hexoses and disaccharides, especially alkyl-glucosides and maltosides, such as hexyl-, heptyl-, octyl-, nonyl- and decyl-beta-D-glucopyranoside or D- maltopyranoside; n-long fatty chain-phosphorylglycerol, such as n- acyl-phosphorylglycerol, especially lauryl-, myristoyl-, oleoyl- or palmitoeloyl- phosphorylglycerol. Surfactant chains are typically chosen to be in a fluid state or at least to be compatible with the maintenance of fluid-chain state in carrier aggregates.

Table 5 lists preferred surfactants in accordance with one embodiment of the disclosure.

Table 5

**Preferred surfactants**

| Fatty chain | | Nonionic surfactants (S) Head / Type / TM | | | | |
|---|---|---|---|---|---|---|
| Name(s) | Length: nr. of double bonds | POE-sorbitan-ester | POE-ether | POE-ester | POE-phenoxy-ether | Selected brandnames |
| | | *Tween* | *Brij, Macrogol* | *Myrj, Nonex* | *Triton* | |
| | C24 | | | | | |
| Behen(o)yl | C22 | | | | | |
| Eruca(o)yl | C22:1-13cis | | | | | |
| Arachin(o)yl | C20 | | | | | |
| Gadolen(o)yl | C20:1-11cis | | | | | |
| Arachidon(o)yl | C20:4-5,8,11,14cis | | | | | |
| Ole(o)yl | C18:1-9cis | Tween 80 | Brij 98 | Simulsol-2599 | TritonX100** | |
| Stear(o)yl | C18 | Tween 60 | | Myrj-52 | | |
| Linol(o)yl | C18:2-9,12cis | | | | | |
| Linole(n/o)yl | C18:3-9,12,15cis | | | | | |
| Palmitole(o)yl | C18:1-9cis | | | | | |
| Palmit(o)yl | C16 | Tween 40 | | NN | | |
| Myrist(o)yl | C14 | | | | | |
| Laur(o)yl | C12 | Tween 20 | Brij 35 | NN | | |
| Capr(o)yl | C10 | | | | | |

**Rel. concentration range L/S (M/M)**

5/1 - 1/1  5/1 - 1/1  5/1 - 1/1  4/1 - 3/2

NN: not readily available in the market but in principle suitable

**Triton is not an oleate, but an octylphenoxy-POE derivative
Myrj-45: Stearoyl-EO8; Myrj-49: Stearoyl-EO20 (not in the market); Myrj-59: Stearoyl-EO100; Myrj-52: Stearoyl-EO40;
**Simulsol-2599 = Macrogol-10-oleate**

Brij-98: Oleoyl-EO20
Brij-35: Lauryl-EO23

The surfactant is a nonionic surfactant. The surfactant may be present in the formulation in about 1% to about 10%, about 1% to about 4%, about 4% to about 7% or about 7% to about 10% by weight. In some emdodiments, the amount of non-ionic surfactant in the formulation is from about 0.2% to about 0.5%. In certain embodiments, the nonionic surfactant is selected from the group consisting of: polyoxyethylene sorbitans (polysobate surfactants), polyhydroxyethylene stearates or polyhydroxyethylene laurylethers (Brij surfactants). In a specific embodiment, the surfactant is a polyoxyethylene-sorbitan- monooleate (e.g. polysorbate 80 or Tween 80). In certain embodiments, the polysorbate can have any chain with 12 to 20 carbon atoms. In certain embodiments, the polysorbate is fluid in the formulation, which may contain one or more double bonds, branching, or cyclo-groups.

## 4.3. FORMULATIONS

In some embodiments, the formulations of the invention comprise only one phospholipid and only one non-ionic surfactant. In other embodiments, the formulations of the invention comprise more than one phospholipid and only one non-ionic surfactant, e.g., two, three, four, or more phospholipids and one non-ionic surfactant. In other embodiments, the formulations of the invention comprise only one phospholipid and more than one non-ionic surfactant, e.g., two, three, four, or more non-ionic surfactants and one phospholipid. In other embodiments, the formulations of the invention comprise more than one phopholipid and more than one non-ionic surfactant, e.g., two, three, four, or more phospholipids and two, three, four, or more non-ionic surfactants.

The formulations of the disclosure may have a range of phospholipid to non-ionic surfactant ratios. The ratios may be expressed in terms of molar terms (mol lipid /mol surfactant). The molar ratio of phospholipid to non-ionic surfactant in the formulations may be from about 1:3 to about 30:1, from about 1:2 to about 30:1, from about 1:1 to about 30:1, from about 5:1 to about 30:1, from about 10:1 to about 30:1, from about 15:1 to about 30:1, or from about 20:1 to about 30:1. In certain embodiments, the molar ratio of lipid to surfactant in the formulations of the invention may be from about 1:2 to about 10:1. In certain embodiments, the ratio may be from about 1:1 to about 2:1, from about 2:1 to about 3:1, from about 3:1 to about 4:1, from about 4:1 to about 5:1 or from about 5:1 to about 10:1. In certain embodiments, the molar ratio may be from about 10:1 to about 30:1, from about 10:1 to about 20:1, from about 10:1 to about 25:1, and from about 20:1 to about 25:1. In specific embodiments, the phospholipid to non-ionic surfactant ratio maybe about 1.0:1.0, about 1.25:1.0, about 1.5:1.0, about 1.75:1.0, about 2.0:1.0, about 2.5:1.0, about 3.0:1.0 or about 4.0:1.0.

The formulations of the invention may also have varying amounts of total amount of the following components: phospholipid and non-ionic surfactant combined (TA). The TA amount may be stated in terms of weight percent of the total composition. In one embodiment, the TA is from about 1% to about 40%, about 5% to about 30%, about 7.5% to about 15%, about 5% to about 10%, about 10% to about 20% or about 20% to about 30%. In specific embodiments, the TA is 8%, 9%, 10%, 15% or 20%.

Selected ranges for total lipid amounts and phospholipid/non-ionic surfactant ratios (mol/mol) for the formulations of the invention are described in Table 6 below:

Table 6: Total Amount and Phospholipid to Non-ionic Surfactant Ratios

| TA (and surfactant) (%) | Lipid/Surfactant (mol/mol) |
|---|---|
| 5 to 10 | 1.0 to 1.25 |
| 5 to 10 | 1.25 to 1.75 |
| 5 to 10 | 1.75 to 2.25 |
| 5 to 10 | 2.25 to 3.00 |
| 5 to 10 | 3.00 to 4.00 |
| 5 to 10 | 4.00 to 8.00 |
| 5 to 10 | 10.00 to 13.00 |
| 5 to 10 | 15.00 to 20.00 |
| 5 to 10 | 20.00 to 22.00 |
| 5 to 10 | 22.00 to 25.00 |
| 10 to 20 | 1.0 to 1.25 |
| 10 to 20 | 1.25 to 1.75 |
| 10 to 20 | 1.25 to 1.75 |
| 10 to 20 | 2.25 to 3.00 |
| 10 to 20 | 3.00 to 4.00 |
| 10 to 20 | 4.00 to 8.00 |
| 10 to 20 | 10.00 to 13.00 |
| 10 to 20 | 15.00 to 20.00 |
| 10 to 20 | 20.00 to 22.00 |
| 10 to 20 | 22.00 to 25.00 |

The formulations of the invention do not comprise a pharmaceutically active agent that has been approved for the treatment of pain associated with osteoarthritis, more specifically deep tissue pain, *e.g.*, from osteoarthritis or joint or muscle pain. The formulations of the invention do not comprise NSAIDs. The formulations of the invention do not comprise opioids. The formulations of the invention do not comprise COX-2 inhibitors. The formulations of the invention do not comprise any analgesic, for example they do not comprise chlorobutanol, ketamine, oxetacaine, propanidide and thiamylal, aminophenol-derivatives, aminophenazol-derivatives, antranilic acid- and arylpropione acid derivatives, azapropazone, bumadizone, chloroquin- and codeine-derivatives, diclophenac, fentanil, ibuprofen, indometacine, ketoprofen, methadone-substances, morazone, morphine and its derivatives, nifenazone, niflumin acid, pentazozine, pethidine, phenazopyridine, phenylbutazone-derivatives (such as 3,5 pyrazolidine dion), pherazone, piroxicam, propoxyphene, propyphenazon, pyrazol- and phenazone-derivatives (aminophenazone, metamizole, monophenylbutazone, oxyphenebutazone, phenylbutazone or phenazonesalyzilate), salicylic acid-derivatives, sulfasalazine, tilidine; acetylsalicylic acid, ethylmorphine, alclofenac, alphaprodine, aminophenazone, anileridine, azapropazone, benfotiamine, benorilate, benzydamine, cetobemidone, chlorophenesincarbamate, chlorothenoxazine, codeine, dextromoramide, dextro-propoxyphene, ethoheptazine, fentanyl, fenyramidol, fursultiamine, flupirtinmaleate, glafenine, hydromorphone, lactylphenetidine, levorphanol, mefenamic acid, meptazonol, methadone, mofebutazone, nalbufine, Na-salt of noramidopyriniummethanesulfonate, nefopam, normethadone, oxycodone, paracetamol, pentazocine, pethidine, phenacetine, phenazocine, phenoperidine, pholcodine, piperylone, piritramide, procaine, propyphenazone, salicylamide, thebacone, tiemonium-odide, or tramadone.

The formulations of the invention may optionally contain one or more of the following ingredients: co-solvents, chelators, buffers, antioxidants, preservatives, microbicides, emollients, humectants, lubricants and thickeners. Preferred amounts of optional components are described in Table 7.

The formulations of the invention may include a buffer to adjust the pH of the aqueous solution to a range from pH 3.5 to pH 9, pH 4 to pH 7.5, or pH 4 to pH 6.5. Examples of buffers include, but are not limited to, acetate buffers, lactate buffers, phosphate buffers, and propionate buffers.

The formulations of the invention are typically formulated in aqueous media. The formulations may be formulated with or without co-solvents, such as lower alcohols.

A "microbicide" or "antimicrobial" agent is commonly added to reduce the bacterial count in pharmaceutical formulations. Some examples of microbicides are short chain alcohols, including ethyl and isopropyl alcohol, chlorbutanol, benzyl alcohol, chlorbenzyl alcohol, dichlorbenzylalcohol, hexachlorophene; phenolic compounds, such as cresol, 4-chloro-m-cresol, p-chloro-m-xylenol, dichlorophene, hexachlorophene, povidon- iodine; parabenes, especially alkyl-parabenes, such as methyl-, ethyl-, propyl-, or butyl- paraben, benzyl paraben; acids, such as sorbic acid, benzoic acid and their salts; quaternary ammonium compounds, such as alkonium salts, e.g., a bromide, benzalkonium salts, such as a chloride or a bromide, cetrimonium salts, e.g., a bromide, phenoalkecinium salts, such as phenododecinium bromide, cetylpyridinium chloride and other salts; furthermore, mercurial compounds, such as phenylmercuric acetate, borate, or nitrate, thiomersal, chlorhexidine or its gluconate, or any antibiotically active compounds of biological origin, or any suitable mixture thereof..

Examples of "antioxidants" are butylated hydroxyanisol (BHA), butylated hydroxytoluene (BHT) and di-tert-butylphenol (LY178002, LY256548, HWA-131, BF-389, CI-986, PD-127443, E-5119, BI-L-239XX, etc.), tertiary butylhydroquinone (TBHQ), propyl gallate (PG), 1-O-hexyl-2,3,5-trimethylhydroquinone (HTHQ); aromatic amines (diphenylamine, p-alkylthio-o-anisidine, ethylenediamine derivatives, carbazol, tetrahydroindenoindol); phenols and phenolic acids (guaiacol, hydroquinone, vanillin, gallic acids and their esters, protocatechuic acid, quinic acid, syringic acid, ellagic acid, salicylic acid, nordihydroguaiaretic acid (NDGA), eugenol); tocopherols (including tocopherols (alpha, beta, gamma, delta) and their derivatives, such as tocopheryl-acylate (e.g., -acetate, - laurate, myristate, -palmitate, -oleate, -linoleate, etc., or an y other suitable tocopheryl- lipoate), tocopheryl-POE-succinate; trolox and corresponding amide and thiocarboxamide analogues; ascorbic acid and its salts, isoascorbate, (2 or 3 or 6)-o-alkylascorbic acids, ascorbyl esters (e.g., 6-o-lauroyl, myristoyl, palmitoyl-, oleoyl, or linoleoyl-L-ascorbic acid, etc.). Also useful are the preferentially oxidised compounds, such as sodium bisulphite, sodium metabisulphite, thiourea; chelating agents, such as EDTA, GDTA, desferral; miscellaneous endogenous defence systems, such as transferrin, lactoferrin, ferritin, cearuloplasmin, haptoglobion, heamopexin, albumin, glucose, ubiquinol-10); enzymatic antioxidants, such as superoxide dismutase and metal complexes with a similar activity, including catalase, glutathione peroxidase, and less complex molecules, such as beta- carotene, bilirubin, uric acid; flavonoids (flavones, flavonols, flavonones, flavanonals, chacones, anthocyanins), N-acetylcystein, mesna, glutathione, thiohistidine derivatives, triazoles; tannines, cinnamic acid, hydroxycinnamatic acids and their esters (coumaric acids and esters, caffeic acid and their esters, ferulic acid, (iso-) chlorogenic acid, sinapic acid); spice extracts (e.g., from clove, cinnamon, sage, rosemary, mace, oregano, allspice, nutmeg); carnosic acid, carnosol, carsolic acid; rosmarinic acid, rosmaridiphenol, gentisic acid, ferulic acid; oat flour extracts, such as avenanthramide 1 and 2; thioethers, dithioethers, sulphoxides, tetralkylthiuram disulphides; phytic acid, steroid derivatives (e.g., U74006F); tryptophan metabolites (e.g., 3-hydroxykynurenine, 3 -hydroxy anthranilic acid), and organochalcogenides.

"Thickeners" are used to increase the viscosity of pharmaceutical formulations to and may be selected from selected from pharmaceutically acceptable hydrophilic polymers, such as partially etherified cellulose derivatives, comprising carboxymethyl-, hydroxyethyl-, hydroxypropyl-, hydroxypropylmethyl- or methyl-cellulose; completely synthetic hydrophilic polymers comprising polyacrylates, polymethacrylates, poly(hydroxyethyl)-, poly(hydroxypropyl)-, poly(hydroxypropylmethyl)methacrylate, polyacrylonitrile, methallylsulphonate, polyethylenes, polyoxiethylenes, polyethylene glycols, polyethylene glycol- lactide, polyethylene glycol-diacrylate, polyvinylpyrrolidone, polyvinyl alcohols, poly(propylmethacrylamide), poly(propylene fumarate-co-ethylene glycol), poloxamers, polyaspartamide, (hydrazine cross-linked) hyaluronic acid, silicone; natural gums comprising alginates, carrageenan, guar-gum, gelatine, tragacanth, (amidated) pectin, xanthan, chitosan collagen, agarose; mixtures and further derivatives or co-polymers thereof and/or other pharmaceutically, or at least biologically, acceptable polymers.

The formulations of the present invention may also comprise a polar liquid medium. The formulations of the invention may be administered in an aqueous medium. The of the present invention may be in the form of a solution, suspension, emulsion, cream, lotion, ointment, gel, spray, film forming solution or lacquer.

In some embodiments, the invention relates to the use of a vesicular formulation as described above for the preparation of a pharmaceutical composition for the treatment of pain associated with osteoarthritis, *e.g.*, deep tissue pain for example from osteoarthritis or other joint or muscle pain. In some embodiments, the invention relates to a vesicular formulation or pharmaceutical composition comprising at least one phospholipid and one nonionic surfactant for the treatment of pain associated with osteoarthritis wherein the formulation or pharmaceutical composition is formulated for topical delivery.

Table 7 lists preferred excipients for the formulation.

Table 7

**Preferred excipients for use in the formulations of the invention**

| Antioxidant | Molar (M) or Rel. w%* | Antibiotic | Molar (M) or Weight-% | Buffer | Molar |
|---|---|---|---|---|---|
| *Primary* | | | | | |
| | | | 30-150 | | 30-150 |
| **Butylated hydroxyanisole, BHA** | 0.1-8 | **Acetate** | mM | **Acetate** | mM |
| **Butylated hydroxytoluene, BHT** | 0.1-4 | **Benzyl alcohol** | 0.1-3 | **Phosphate** | 10-50 mM |
| | | | | | 30-150 |
| Thymol | 0.1-1 | **Butylparabene** | 0.1-3 | Triethanolamine.HCL | mM |
| **Metabisulphite** (MW = 190.1) | 1-5 mM | **Ethylparabene** | 0.1-3 | | |
| Bisulphite | 1-5 mM | **Imidurea** (MW = 388.30) | 0.1-1 | | |
| | | **Dimethoxane** (MW = 174.2) | 0.03-0.1 | | |
| Thiourea (MW = 76.12) | 1-10 mM | **Methylparabene** | 0.1-5 | | |
| Monothioglycerol (MW = 108.16) | 1-20 mM | | | | |
| Propyl gallate (MW = 212.2) | 0.02-0.2 | **Phenoxyethanol** | 0.1-5 | | |
| Ascorbate (MW = 175.3+ ion) | 1-10 mM | Benzalkonium chloride | 0.01-0.2 | | |
| Palmityl-ascorbate | 0.01-1 | Benzethonium chloride | 0.01-0.1 | | |
| Tocopherol-PEG | 0.5-5 | Phenol | 0.05-2 | | |
| *Secondary (chelator)* | | Phenylethyl alcohol | 0.1-1 | | |
| **EDTA** (MW = 292) | 1-10 mM | Thimerosal | 0.005-0.1 | | |
| **EGTA** (MW = 380.35) | 1-10 mM | | | | |
| **Desferal** (MW = 656.79) | 0.1-5 mM | | | | |

*As percentage of Total Lipid quantity*

EGTA= Ethylene glycol-bis-(2-aminoethyl)-N,N,N', N'-tetraacetic acid

EDTA = Ethylenedioxy-diethylene-dinitrilo-tetraacetic acid

## 4.4. <u>VESICULAR FORMULATIONS</u>

While not to be limited to any mechanism of action or any theory, the formulations of the invention may form vesicles or ESAs characterized by their adaptability, deformability, or penetrability.

The term vesicle or aggregate "adaptability" which governs the "tolerable surface curvature" is defined as the ability of a given vesicle or aggregate to change easily its properties, such as shape, elongation ratio, and surface to volume ratio. The vesicles of this invention may be characterized by their ability to adjust the aggregates' shape and properties to the anisotropic stress caused by pore crossing. Sufficient adaptability implies that a vesicle or an aggregate can sustain different unidirectional forces or stress, such as one caused by pressure, without extensive fragmentation, which defines a "stable" aggregate. If an aggregate passes through a barrier fulfilling this condition the terms "adaptability" and (shape) "deformability" plus "permeability" are essentially equivalent. A "barrier" in the context of this invention is (as in, for example, EP 0 475 160 and WO 98/17255) a body with through-extending narrow pores, such narrow pores having a radius which is at least 25% smaller than the radius of the ESAs (considered as spherical) before said ESAs permeate through such pores.

The term "narrow" used in connection with a pore implies that the pore radius is significantly, typically at least 25%, smaller than the radius of the entity tested with regard to its ability to cross the pore. The necessary difference typically should be greater for the narrower pores. Using 25% limit is therefore quite suitable for > 150 nm diameter whereas >100% difference requirement is more appropriate for the smaller systems, e.g., with <50 nm diameter. For diameters around 20 nm, aggregate diameter difference of at least 200% is often required.

The term "semipermeable" used in connection with a barrier implies that a solution can cross transbarrier openings whereas a suspension of non-adaptable aggregates (large enough for the above definition of "narrow" pores to apply) cannot. Conventional lipid vesicles (liposomes) made from any common phosphatidylcholine in the gel lamellar phase or else from any biological phosphatidylcholine/cholesterol 1/1 mol/mol mixture or else comparably large oil droplets, all having the specified relative diameter, are three examples of such non-adaptable aggregates.

The term "stable" means that the tested aggregates do not change their diameter spontaneously or under the transport related mechanical stress (e.g. during passage through a semipermeable barrier) unacceptably, which most often means only to a pharmaceutically acceptable degree. A 20-40% change is normally considered acceptable; the halving or doubling of aggregate diameter is borderline and a greater change in diameter is typically unacceptable. Alternatively and very conveniently, the change in aggregate diameter resulting from pore crossing under pressure is used to assess system stability; the same criteria are then applied as for "narrow" pores, mutatis mutandis. To obtain the correct value for aggregate diameter change, a correction for flux/vortex effects may be necessary. These procedures are described in greater detail in the publications of the applicant in Cevc et. al., Biochim. Biophys. Acta 2002; 1564:21-30.

Non-destructing passage of ultradeformable, mixed lipid aggregates through narrow pores in a semi-permeable barrier is thus diagnostic of high aggregate adaptability. If pore radius is two times smaller than the average aggregate radius the aggregate must change its shape and surface-to-volume ratio at least 100% to pass without fragmentation through the barrier. An easy and reversible change in aggregate shape inevitably implies high aggregate deformability and requires large surface-to-volume ratio adaptation. A change in surface-to-volume ratio per se implies: a) high volume compressibility, e.g. in the case of compact droplets containing material other than, and immiscible with, the suspending fluid; b) high aggregate membrane permeability, e.g. in the case of vesicles that are free to exchange fluid between inner and outer vesicle volume.

The vesicles or ESAs of the present invention have "adaptability" that can be assessed using the following method: 1) measure the flux ($j_a$) of the aggregate or ESA suspension through a semi-permeable membrane (e.g., gravimetrically) for different transport-driving trans barrier pressures ($\Delta p$); 2) calculate the pressure dependence of barrier penetratability P for the suspension by dividing each measured flux value by the corresponding pressure value: $P(\Delta p)$ = $j_a(\Delta p)/\Delta p$; 3) monitor the ratio of final and starting vesicle diameter 2 $r_{ves}(\Delta p)/2$ $r_{ves,0}$ (*e.g.* by dynamic light scattering), wherein 2 $r_{ves}(\Delta p)$ is the vesicle diameter after semi-permeable barrier passage driven by $\Delta p$ and 2 $r_{ves,0}$ is the starting vesicle diameter, and if necessary make corrections for the flow-effects; and 4) aligh both data sets $P(\Delta p)$ vs. $r_{ves}(\Delta p)/r_{ves,0}$ to determine the co-existence range for high aggregate adaptability and stability.

It is also useful, but not essential, to parameterize experimental penetratability data within the framework of Maxwell-approximation in terms of the necessary pressure value p* and in terms of maximum penetratability value $P_{max}$. It is plausible to sum-up all the contributions to a moving aggregate energy (deformation energy/ies, thermal energy, the shearing work, etc.) into a single, total energy. The equilibrium population density of aggregate's energetic levels then may be taken to correspond to Maxwell's distribution. All aggregates with a total energy greater than the activation energy, E *f* $E_A$, are finally concluded to penetrate the barrier. The pore-crossing probability for such aggregates is then given by the following formula, where *e* is dimensionless aggregate energy units of the activation energy $E_A$:

$$P(e) = 1 - erf\left(\sqrt{\frac{1}{e}}\right) + \sqrt{\frac{4}{\pi e}} \cdot \exp\left[-\frac{1}{e}\right]$$

It is therefore plausible to represent barrier penetratibility of a given suspension as a function of transport driving pressure by the following formula, where $P_{max}$ is the maximum possible penetratability of a given barrier (for the aggregates with zero transport resistance this penetrability is identical to the penetrability of the suspending medium flux), and p* is an adjustable parameter that describes the pressure sensitivity, and thus the transport resistance, of the tested system (for barriers with a fixed pore radius this sensitivity is a function of aggregate properties solely; for non-interacting particles the sensitivity is dominated by aggregate adaptability, allowing to make the assumption: $a_a$ proportional to 1/p*

$$P(p) = p_{\max} \cdot \left\{ 1 - erf\left(\sqrt{\frac{p^*}{p}}\right) + \sqrt{\frac{4p^*}{\pi p}} \cdot \exp\left[-\frac{p^*}{p}\right]\right\}$$

Other methods of testing deformability and adaptability which may be used to characterize the compsitions of the invention are set forth, for example, in U.S. Patent Application Publication Nos. 2004/0071767 and 2004/0105881.

### 4.5. METHODS OF ADMINISTRATION / TREATMENT

In another embodiment, the present disclosure provides a formulation according to the claims, wherein the formulation is administered to a subject in need thereof. The formulation comprises optionally buffers, antioxidants, preservatives, microbicides, antimicrobials, emollients, co-solvents, and/or thickeners.

### 4.6. PACKAGES

In another embodiment, the invention provides a pharmaceutical package or kit comprising one or more containers filled with the formulation of the invention, and instructions for administration of the formulation to a patient or subject in need thereof for the treatment of pain associated with osteoarthritis. The formulation comprises one or more phospholipids and one or more non-ionic surfactants. The formulation may comprise a lysophospholipid. The formulation does not comprise a non-lipid non-surfactacnt pharmaceutically active agent that has been approved for the treatment of pain associated with osteoarthritis. In various embodiments, the container comprises a formulation formulated as a suspension, emulsion, gel, cream, lotion, spray, film forming solution or lacquer. The disclsoure provides packages or kits that can be used in any of the above-described methods.

### 5. EXAMPLES

### 5.1 Example 1: Example Formulations

The following exemplary formulations for topical application may be prepared by the following procedure:

1. Organic phase production, which contains all lipophilic excipients

The organic phase is produced by weighing the phospholipid, the non-ionic surfactant, any additional lipophilic excipients into suitable containers followed by mixing these components into anoptically isotropic phase which appears as a clear solution. During mixing, the organic phase will be heated up, but temperature must not rise above 45°C.

2. Aqueous phase production

The aqueous phase is prepared by weighing the non-lipophilic components and water, which serves as solvent, into suitable containers and then mixing these components into a clear solution. During mixing, the temperature will be elevated to 40°C.

3. Production of a concentrated intermediate by combination of both phases

The isotropic organic phase and the clear aqueous phase are combined under stirring in a suitable vessel. Before and during the combination the temperature of both phases must be kept between 35°C and 45°C. The resulting intermediate is homogenised mechanically at 40°C. Before starting homogenisation, the pressure in the production vessel is lowered to - 0.08 MPa. The desired average carrier size is typically reached after 10 minutes of homogenisation.

Three process parameters must be controlled carefully during the production of the concentrated intermediate: temperature, homogeniser circulation velocity, and overall processing time.

4. Production of the final bulk product by mixing the concentrated intermediate with dilution buffer.

The concentrated intermediate is diluted with the dilution buffer to the intended final concentration. The mixture is carefully stirred in the mixing vessel at 20°C to homogeneity.

Table 8 describes the amounts of surfactant and lipids, and other excipients in the tranfersome formulations, described in terms of the percent of the total amount of formulation.

**TABLE 8: Preferred Formulations**

**Table 8A: This table lists the relative amounts of each of the components of Preferred Formulations**

| | Lipid mg/g | Surfactant mg/g (1 to 10% by wt.) | Buffer (pH 4-7.5) | Antimicrobials (0-10 mg/g) | Antioxidants (0-10mg/g) | Emollient (0-50 mg/g) | Other (0-50mg/g) | Chelator (0-25mg/g) |
|---|---|---|---|---|---|---|---|---|
| 1 | 47.944 | 42.056 | 4 | 5,000 | 0.700 | 30.000 | 30.000 | 3.000 |
| 2 | 53.750 | 31.250 | 4 | 5.000 | 0.700 | 30.000 | 15.000 | 3.000 |
| 3 | 90.561 | 79.439 | 4 | 5.000 | 0.700 | 30.000 | 30.000 | 3.000 |
| 4 | 47.944 | 42.056 | 5 | 5.000 | 0.700 | 30.000 | 30.000 | 3.000 |
| 5 | 50.607 | 44.393 | 5 | 5.000 | 0.700 | 0.000 | 10.000 | 3.000 |
| 6 | 90.561 | 79.439 | 5 | 5.000 | 0.700 | 30.000 | 30.000 | 3.000 |
| 7 | 49.276 | 43.224 | 6.5 | 5.000 | 0.700 | 30.000 | 30.000 | 3.000 |
| 8 | 53.750 | 31.250 | 6.5 | 5.000 | 0.200 | 30.000 | 0.000 | 3.000 |
| 9 | 90.561 | 79.439 | 6.5 | 5.000 | 0.200 | 30.000 | 20.000 | 3.000 |
| 10 | 41.351 | 48.649 | 4 | 5.000 | 0.200 | 30.000 | 30.000 | 3.000 |
| 11 | 47.882 | 37.118 | 4 | 5.000 | 0.200 | 0.000 | 30.000 | 3.000 |
| 12 | 95.764 | 74.236 | 4 | 5.000 | 0.200 | 30.000 | 30.000 | 3.000 |
| 13 | 65.676 | 24.324 | 5 | 5.000 | 0.200 | 0.000 | 25.000 | 3.000 |
| 14 | 62.027 | 22.973 | 5 | 5.000 | 0.200 | 0.000 | 30.000 | 3.000 |
| 15 | 124.054 | 45.946 | 5 | 5.000 | 0.200 | 15.000 | 36.510 | 3.000 |
| 16 | 62.687 | 32.313 | 6.5 | 5.000 | 0.200 | 15.000 | 0.000 | 3.000 |
| 17 | 41.853 | 43.147 | 6.5 | 5.000 | 0.200 | 30.000 | 30.000 | 3.000 |
| 18 | 95.764 | 74.236 | 6.5 | 5000 | 0.200 | 0.000 | 30.000 | 3.000 |
| 19 | 47.882 | 37.118 | 6.5 | 5.000 | 0.200 | 0.000 | 0.000 | 3.000 |
| 20 | 45.000 | 45.000 | 6.5 | 5.000 | 0.200 | 0.000 | 0.000 | 1.000 |
| 21 | 31.935 | 58.065 | 5 | 5.000 | 0.200 | 30.000 | 15.000 | 3.000 |
| 22 | 42.500 | 42.500 | 6.5 | 5.000 | 0.200 | 30.000 | 0.000 | 3.000 |
| 23 | 38.276 | 51.724 | 4 | 5.000 | 0.200 | 0.000 | 36.510 | 3.000 |
| 24 | 42.500 | 42.500 | 4 | 5.000 | 0.200 | 0.000 | 15.000 | 3.000 |
| 25 | 85.000 | 85.000 | 4 | 5.000 | 0.200 | 30.000 | 30.000 | 3.000 |
| 26 | 38.276 | 51.724 | 5 | 5.000 | 0.200 | 30.000 | 0.000 | 1.000 |
| 27 | 36.429 | 48.571 | 5 | 5.000 | 0.200 | 30.000 | 30.000 | 3.000 |
| 28 | 72.299 | 97.701 | 5 | 5.000 | 0.200 | 30.000 | 15.000 | 3.000 |
| 29 | 46.250 | 46.250 | 6.5 | 5.000 | 0.700 | 0.000 | 20.000 | 3.000 |
| 30 | 38.804 | 46.196 | 6.5 | 5.000 | 0.700 | 15.000 | 30.000 | 3.000 |
| 31 | 36.667 | 33.333 | 6.5 | 5.000 | 0.700 | 30.000 | 10.000 | 3.000 |

EP 2 437 726 B1

(continued)

| | Lipid mg/g | Surfactant mg/g (1 to 10% by wt.) | Buffer (pH 4-7.5) | Antimicrobials (0-10 mg/g) | Antioxidants (0-10mg/g) | Emollient (0-50 mg/g) | Other (0-50mg/g) | Chelator (0-25mg/g) |
|---|---|---|---|---|---|---|---|---|
| 32 | 66.667 | 23.333 | 4 | 5.000 | 0.200 | 0.000 | 0.000 | 3.000 |
| 33 | 45.833 | 41.667 | 4 | 5.000 | 0.200 | 30.000 | 0.000 | 3.000 |
| 34 | 31.957 | 38.043 | 4 | 5.000 | 0.200 | 0.000 | 30.000 | 3.000 |
| 35 | 47.143 | 42.857 | 5 | 5.000 | 0.200 | 30.000 | 25.000 | 1.000 |
| 36 | 96.905 | 88.095 | 5 | 5.000 | 0.200 | 30.000 | 20.000 | 3.000 |
| 37 | 31.957 | 38.043 | 5 | 5.000 | 0.200 | 0.000 | 30.000 | 3.000 |
| 38 | 35.455 | 54.545 | 6.5 | 5.000 | 0.700 | 30.000 | 0.000 | 3.000 |
| 39 | 84.457 | 100.543 | 6.5 | 5.000 | 0.700 | 30.000 | 30.000 | 3.000 |
| 40 | 89.048 | 80.952 | 6.5 | 5.000 | 0.700 | 30.000 | 30.000 | 3.000 |
| 41 | 41.087 | 48.913 | 4 | 5.000 | 0.700 | 30.000 | 36.510 | 3.000 |
| 42 | 45.280 | 39.720 | 4 | 5.000 | 0.700 | 0.000 | 0.000 | 3.000 |
| 43 | 107.500 | 62.500 | 4 | 5.000 | 0.700 | 30.000 | 30.000 | 3.000 |
| 44 | 77.243 | 67.757 | 4 | 5.000 | 0.700 | 0.000 | 15.000 | 3.000 |
| 45 | 45.280 | 39.720 | 5 | 5.000 | 0.700 | 0.000 | 20.000 | 3.000 |
| 46 | 90.561 | 79.439 | 5 | 5.000 | 0.700 | 0.000 | 30.000 | 3.000 |
| 47 | 47.944 | 42.056 | 5 | 5.000 | 0.700 | 0.000 | 10.000 | 3.000 |
| 48 | 50.607 | 44.393 | 5.5 | 5.000 | 0.700 | 30.000 | 0.000 | 1.000 |
| 49 | 107.500 | 62.500 | 5.5 | 5.000 | 0.700 | 30.000 | 0.000 | 3.000 |
| 50 | 47.944 | 42.056 | 5.5 | 5.000 | 0.700 | 30.000 | 30.000 | 3.000 |
| 51 | 46.364 | 38.636 | 4 | 5.000 | 0.200 | 30.000 | 25.000 | 3.000 |
| 52 | 46.364 | 38.636 | 4 | 5.000 | 0.200 | 0.000 | 20.000 | 3.000 |
| 53 | 46.098 | 43.902 | 5 | 5.000 | 0.200 | 15.000 | 30.000 | 3.000 |
| 54 | 43.537 | 41.463 | 5 | 5.000 | 0.200 | 30.000 | 0.000 | 3.000 |
| 55 | 45.000 | 45.000 | 5 | 5.000 | 0.200 | 0.000 | 30.000 | 3.000 |
| 56 | 59.492 | 30.508 | 6.5 | 5.000 | 0.200 | 30.000 | 30.000 | 3.000 |
| 57 | 39.054 | 45.946 | 6.5 | 5.000 | 0.200 | 0.000 | 0.000 | 3.000 |
| 58 | 35.854 | 34.146 | 6.5 | 5.000 | 0.200 | 30.000 | 0.000 | 3.000 |
| 59 | 50.000 | 40.000 | 6.5 | 5.000 | 0.700 | 30.000 | 30.000 | 3.000 |
| 60 | 38.571 | 51.429 | 6.5 | 5.000 | 0.700 | 30.000 | 30.000 | 3.000 |
| 61 | 41.954 | 50.546 | 6.5 | 5.000 | 0.700 | 30.000 | 30.000 | 3.000 |
| 62 | 42.632 | 47.368 | 6.5 | 5.000 | 0.700 | 30.000 | 30.000 | 3.000 |
| 63 | 46.098 | 43.902 | 6.5 | 5.000 | 0.700 | 30.000 | 30.000 | 3.000 |

| | Lipid mg/g | Surfactant mg/g (1 to 10% by wt.) | Buffer (pH 4-7.5) | Antimicrobials (0-10 mg/g) | Antioxidants (0-10mg/g) | Emollient (0-50 mg/g) | Other (0-50mg/g) | Chelator (0-25mg/g) |
|---|---|---|---|---|---|---|---|---|
| 64 | 39.721 | 50.279 | 6.5 | 5.000 | 0.700 | 30.000 | 30.000 | 3.000 |
| 65 | 44.198 | 50.802 | 6.5 | 5.000 | 0.700 | 30.000 | 30.000 | 3.000 |
| 66 | 46.453 | 51.047 | 6.5 | 5.000 | 0.700 | 30.000 | 30.000 | 3.000 |
| 67 | 51.221 | 43.779 | 6.5 | 5.000 | 0.700 | 30.000 | 30.000 | 3.000 |
| 68 | 54.167 | 43.333 | 6.5 | 5.000 | 0.700 | 30.000 | 30.000 | 3.000 |
| 69 | 66.440 | 23.560 | 6.5 | 5.000 | 0.700 | 30.000 | 30.000 | 3.000 |
| 70 | 66.440 | 23.560 | 6.5 | 5.000 | 0.700 | 30.000 | 30.000 | 3.000 |
| 71 | 66.440 | 23.560 | 6.5 | 5.000 | 0.700 | 30.000 | 30.000 | 3.000 |
| 72 | 40,000 | 50.000 | 6.5 | 5.000 | 0.700 | 30.000 | 30.000 | 3.000 |
| 73 | 40.000 | 50.000 | 6.5 | 5.000 | 0.700 | 30.000 | 30.000 | 3.000 |
| 74 | 40.000 | 50.000 | 5.5 | 0.000 | 0.700 | 30.000 | 30.000 | 3.000 |
| 75 | 40.000 | 50.000 | 6.5 | 5.000 | 0.700 | 30.000 | 30.000 | 3.000 |
| 76 | 40.000 | 50.000 | 6.5 | 5.000 | 0.700 | 30.000 | 30.000 | 3.000 |
| 77 | 40.000 | 50.000 | 6.5 | 5.000 | 0.700 | 30.000 | 30.000 | 3.000 |
| 78 | 66.440 | 23.560 | 6.5 | 5.000 | 0.700 | 30.000 | 30.000 | 3.000 |
| 79 | 66.440 | 23.560 | 6.5 | 5.000 | 0.700 | 30.000 | 30.000 | 3.000 |
| 80 | 40.000 | 50.000 | 5.5 | 0.000 | 0.700 | 30.000 | 30.000 | 3.000 |
| 81 | 40.000 | 50.000 | 5.5 | 5.000 | 0.700 | 30.000 | 30.000 | 3.000 |
| 82 | 44.444 | 55.556 | 5.5 | 5.000 | 0.700 | 30.000 | 30.000 | 3.000 |
| 83 | 66.440 | 23.560 | 5.5 | 5.000 | 0.700 | 30.000 | 30.000 | 3.000 |
| 84 | 54.000 | 36.000 | 4 | 5.000 | 0.700 | 30.000 | 30.000 | 3.000 |
| 85 | 50.000 | 40.000 | 4 | 5.000 | 0.700 | 30.000 | 30.000 | 3.000 |
| 86 | 48.611 | 38.889 | 4 | 5.000 | 0.700 | 30.000 | 30.000 | 3.000 |
| 87 | 46.575 | 38.425 | 4 | 5.000 | 0.700 | 30.000 | 30.000 | 3.000 |
| 88 | 46.575 | 38.425 | 4 | 5.000 | 0.700 | 30.000 | 30.000 | 3.000 |
| 89 | 46.575 | 38.425 | 4 | 5.000 | 0.700 | 30.000 | 30.000 | 3.000 |
| 90 | 50.000 | 40.000 | 4.5 | 5.000 | 0.700 | 30.000 | 30.000 | 3.000 |
| 91 | 94.444 | 75.556 | 4 | 5.000 | 0.700 | 30.000 | 30.000 | 3.000 |
| 92 | 46.712 | 38.288 | 4 | 5.000 | 0.700 | 30.000 | 30.000 | 3.000 |
| 93 | 48.889 | 39.111 | 4 | 5.000 | 0.700 | 30.000 | 30.000 | 3.000 |
| 94 | 39.721 | 50.279 | 6.5 | 5.000 | 0.700 | 3.000 | 30.000 | 3.000 |
| 95 | 90.000 | 0.000 | 6.5 | 5.000 | 0.700 | 30.000 | 30.000 | 3.000 |

| | Lipid mg/g | Surfactant mg/g (1 to 10% by wt.) | Buffer (pH 4-7.5) | Antimicrobials (0-10 mg/g) | Antioxidants (0-10mg/g) | Emollient (0-50 mg/g) | Other (0-50mg/g) | Chelator (0-25mg/g) |
|---|---|---|---|---|---|---|---|---|
| 96 | 68.700 | 8.500 | 7.5 | 5.000 | 0.700 | 30.000 | 36.510 | 1.000 |
| 97 | 71.460 | 4.720 | 7.8 | 5.000 | 0.700 | 50.000 | 35.000 | 3.000 |
| 99 | 71.460 | 4.720 | 7.8 | 5.000 | 0.700 | 50.000 | 15.000 | 3.000 |
| 98 | 71.460 | 4.720 | 7.8 | 0.000 | 0.700 | 50.000 | 15.000 | 3.000 |
| 100 | 71.460 | 4.720 | 7.8 | 0.000 | 0.700 | 50.000 | 35.000 | 3.000 |
| 101 | 46.575 | 38.425 | 4 | 0.000 | 0.700 | 0.000 | 0.000 | 3.000 |
| 102 | 46.575 | 38.425 | 4 | 0.000 | 0.700 | 0.000 | 0.000 | 3.000 |
| 103 | 54.643 | 30.357 | 4 | 5.000 | 0.700 | 0.000 | 0.000 | 3.000 |
| 104 | 39.72 | 50.279 | 6.5 | 5.000 | 0.700 | 30.000 | 30.000 | 3.000 |
| 105 | 90.00 | | 6.5 | 5.000 | 0.700 | 30.000 | 30.000 | 3.000 |
| 106 | 46.57 | 38.425 | 4 | | 0.700 | | | 3.000 |
| 107 | 46.75 | 38.425 | 4 | | 0.700 | | | 3.000 |
| 108 | 54.64 | 30.357 | 4 | | 0.700 | | | 3.000 |
| 109 | 46.364 | 38.836 | 4 | 5.000 | 0.200 | 30.000 | 25.000 | 3.000 |
| 110 | 46.364 | 38.636 | 4 | 5.000 | 0.200 | 0.000 | 20.000 | 3.000 |
| 111 | 46.098 | 43.902 | 5 | 5.000 | 0.200 | 15.000 | 30.000 | 3.000 |
| 112 | 43.537 | 41.463 | 5 | 5.000 | 0.200 | 30.000 | 0.000 | 3.000 |
| 113 | 45.000 | 45.000 | 5 | 5.000 | 0.200 | 0.000 | 30.000 | 3.000 |
| 114 | 59.492 | 30.508 | 6.5 | 5.000 | 0.200 | 30.000 | 30.000 | 3.000 |
| 115 | 39.054 | 45.946 | 6.5 | 5.000 | 0.200 | 0.000 | 0.000 | 3.000 |
| 116 | 35.854 | 34.146 | 6.5 | 5.000 | 0.200 | 30.000 | 0.000 | 3.000 |
| 117 | 50.000 | 40.000 | 6.5 | 5.000 | 0.700 | 30.000 | 30.000 | 3.000 |
| 118 | 38.571 | 51.429 | 6.5 | 5.000 | 0.700 | 30.000 | 30.000 | 3.000 |
| 119 | 41.954 | 50.546 | 6.5 | 5.000 | 0.700 | 30.000 | 30.000 | 3.000 |
| 120 | 42.632 | 47.368 | 6.5 | 5.000 | 0.700 | 30.000 | 30.000 | 3.000 |
| 121 | 46.098 | 43.902 | 6.5 | 5.000 | 0.700 | 30.000 | 30.000 | 3.000 |
| 122 | 39.721 | 50.279 | 6.5 | 5.000 | 0.700 | 30.000 | 30.000 | 3.000 |
| 123 | 44.198 | 50.802 | 6.5 | 5.000 | 0.700 | 30.000 | 30.000 | 3.000 |
| 124 | 46.453 | 51.047 | 6.5 | 5.000 | 0.700 | 30.000 | 30.000 | 3.000 |
| 125 | 51.221 | 43.779 | 6.5 | 5.000 | 0.700 | 30.000 | 30.000 | 3.000 |
| 126 | 54.167 | 43.333 | 6.5 | 5.000 | 0.700 | 30.000 | 30.000 | 3.000 |
| 127 | 66.440 | 23.560 | 6.5 | 5.000 | 0.700 | 30.000 | 30.000 | 3.000 |

(continued)

| | Lipid mg/g | Surfactant mg/g (1 to 10% by wt.) | Buffer (pH 4-7.5) | Antimicrobials (0-10 mg/g) | Antioxidants (0-10mg/g) | Emollient (0-50 mg/g) | Other (0-50mg/g) | Chelator (0-25mg/g) |
|---|---|---|---|---|---|---|---|---|
| 128 | 66.440 | 23.560 | 6.5 | 5.000 | 0.700 | 30.000 | 30.000 | 3.000 |
| 129 | 66.440 | 23.560 | 6.5 | 5.000 | 0.700 | 30.000 | 30.000 | 3.000 |

**Table 8B: The table lists the specific components of the formulas listed above.**

| Formula | Lipid | Surfactant | Buffer | Antimicrobial | Antioxidants | Emollient | Chelator | Other |
|---|---|---|---|---|---|---|---|---|
| 1-4 | Sphingomyelin, *e.g.*, brain | Tween 80 | Lactate | Benzyl alcohol or paraben | BHT (0.200) sodium metabisulfite (0.500) | Glycerol | EDTA | Ethanol |
| 5-7 | Sphingomyelin, lauroyl | Brij 98 | Acetate | Benzyl alcohol or paraben | BHT (0.200) sodium metabisulfite (0500) | Glycerol | EDTA | Ethanol |
| 8-12 | Phosphatidyl choline + Phosphatidylglycerol | Brij 98 | Phosphate | Benzyl alcohol or paraben | HTHQ | Glycerol | EDTA | Ethanol |
| 13-16 | phosphatidyl choline + phosphatidylinositol | Span 20 | Acetate | Benzyl alcohol or paraben | HTHQ | Glycerol | EDTA | Ethanol |
| 17-18 | Phosphatidyl choline + phosphatidic acid | Tween 80 | Phosphate | Benzyl alcohol or paraben | BHT | Glycerol | EDTA | Ethanol |
| 19 | Phosphatidyl choline + phosphatidic acid | Brij 98+ Tween 80 | Phosphate | Benzyl alcohol, or paraben | BHT | Glycerol | EDTA | Ethanol |
| 20 | phosphatidyl cholines + phosphatidic acid | Span 20 + Tween 80 | Phosphate | Benzyl alcohol or paraben | BHT | Glycerol | EDTA | Ethanol |
| 21 | Phosphatidyl cholines | Cremophor + Span 20 | Lactate | Thimerosal | BHA | Glycerol | EDTA | Ethanol |
| 22 | Phosphatidyl choline | Cremophor + Tween 80 | Lactate | Thimerosal | BHA | Glycerol | EDTA | Ethanol |
| 23-28 | Phosphatidyl cholines | Cremophor | Lactate | Thimerosal | BHA | Glycerol | EDTA | Ethanol |
| 29-30 | Phosphatidyl ethanolamine | Tween 80 | Phosphate | Thimerosal | BHT (0,200) sodium metabisulfite (0.500) | glycerol | EDTA | Ethanol |
| 31 | Phosphatidyl ethanolamines | (Brij 98 + Tween 80 | Phosphate | Thimerosal | BHT (0.200) sodium metabisulfite (0.500) | Glycerol | EDTA | Ethanol |
| 32 | Phosphatidyl glycerol | Cremophor + Brij 98 | Acetate | Benzyl alcohol or paraben | BHT | Glycerol | EDTA | Ethanol |
| 33-37 | Phosphatidyl glycerol | Brij 98 | Acetate | Benzyl alcohol or paraben | BHT | Glycerol | EDTA | Ethanol |
| 39-40 | Phosphatidyl ethanolamine | Cremophor | phosphate | Benzyl alcohol or paraben | BHT (0.200) sodium metabisulfite (0.500) | glycerol | EDTA | Ethanol |

| Formula | Lipid | Surfactant | Buffer | Antimicrobial | Antioxidants | Emollient | Chelator | Other |
|---------|-------|-----------|--------|---------------|--------------|-----------|----------|-------|
| 41-47 | Phosphatidyl glycerol | Tween 80 | Propionate | Benzyl alcohol or paraben | BHT (0.200) sodium metabisulfite (0.500) | Glycerol | EDTA | Ethanol |
| 48-50 | Phosphatidyl serine | Brij 99 | Phosphate | Thimerosal | BHT (0.200) sodium metabisulfite (0.500) | Glycerol | EDTA | Ethanol |
| 51-58 | Phosphatidyl glycerol | Brij 98 | Acetate | Benzyl alcohol or paraben | BHT | glycerol | EDTA | Ethanol |
| 59-68 | Phosphatidyl choline | Tween 80 | Phosphate | Benzyl alcohol or paraben | BHT (0.200) sodium metabisulfite (0.500) | glycerol | EDTA | Ethanol |
| 69-71 | Phosphatidy choline | Brij 98 | Phosphate | Benzyl alcohol or paraben | BHT (0.200) sodium metabisulfite (0.500) | glycerol | EDTA | Ethanol |
| 72-73 | Phosphatidyl choline | Tween 80 | Phosphate | Benzyl alcohol or paraben | BHT (0.200) sodium metabisulfite (0.500) | Glycerol | EDTA | Ethanol |
| 74 | Phosphatidyl choline | Tween 80 | Acetate | | BHT (0.200) sodium metabisulfite (0.500) | Glycerol | EDTA | Ethanol |
| 75 | Phosphatidyl cholines | Tween 80 | Phosphate | Benzyl alcohol or paraben | BHT (0.200) sodium metabisulfite (0.500) | Glycerol | EDTA | Methanol |
| 76 | Phosphatidyl choline | Brij 98 | Phosphate | Benzalkonium chloride | BHT (0.200) sodium metabisulfite (0.500) | glycerol | EDTA | Ethanol |
| 77 | Phosphatidyl choline | Tween 80 | Phosphate | Benzyl alcohol or paraben | BHT (0.200) sodium metabisulfite (0.500) | Glycerol | EDTA | Ethanol |
| 78 | Phosphatidyl choline | Brij 98 | Phosphate | Benzalkonium chloride | BHT (0.200) sodium metabisulfite (0.500) | Glycerol | EDTA | Ethanol |
| 79 | Phosphatidyl choline | Brij 98 | Phosphate | Benzyl alcohol or paraben | BHT (0.200) sodium metabisulfite (0.500) | Glycerol | EDTA | Ethanol |
| 80 | Phosphatidyl choline | Tween 80 | Acetate | | BHT (0.200) sodium metabisulfite (0.500) | glycerol | EDTA | Ethanol |
| 81 | Phosphatidyl choline | Tween 80 | Acetate | Benzyl alcohol or paraben | BHT (0.200) sodium metabisulfite (0.500) | Glycerol | EDTA | Ethanol |
| 82-93 | Phosphatidyl choline | Tween 80 | Acetate | Benzyl alcohol or paraben | BHT (0.200) sodium metabisulfite (0.500) | glycerol | EDTA | Ethanol |

EP 2 437 726 B1

(continued)

| Formula | Lipid | Surfactant | Buffer | Antimicrobial | Antioxidants | Emollient | Chelator | Other |
|---|---|---|---|---|---|---|---|---|
| 84-88 | Phosphatidyl choline | Tween 80 | Acetate | Benzyl alcohol or paraben | BHA (0.200) sodium metabisultite (0.500) | Glycerol | EDTA | Ethanol |
| 89 | Phosphatidyl choline | Tween 80 | Acetate | Benzyl alcohol or paraben | BHT (0.200) sodium metabisulfite (0.500) | glycerol | EDTA | Ethanol |
| 90-93 | Phosphatidyl choline | Tween 80 | Acetate | Benzyl alcohol or paraben | BHT (0.200) sodium metabisulfite (0.500) | Glycerol | EDTA | Ethanol |
| 94-96 | Phosphatidyl choline | Tween 80 | Phosphate | Benzyl alcohol or paraben | BHT (0.200) sodium metabisulfite (0.500) | Glycerol | EDTA | Ethanol |
| 97.98 | Phosphatidyl choline | Tween 80 | Phosphate | Benzyl alcohol or paraben | BHT (0.200) sodium metabisulfite (0.500) | Glycerol | EDTA | Ethanol |
| 99-100 | Phosphatidyl choline | Tween 80 | Phosphate | | BHT (0.200) sodium metabisulfite (0.500) | Glycerol | EDTA | Ethanol |
| 101-103 | Phosphatidyl choline | Tween 80 | Acetate | | BHT (0.200) sodium metabisulfite (0.500) | | EDTA | |
| 104 | Phosphatidyl choline | Tweet 80 | Phosphate | Benzyl alcohol or paraben | BHT (0.200) sodium metabisulfite (0.500) | Glycerol | EDTA | Ethanol |
| 105 | Phosphatidyl choline | | Phosphate | Benzyl alcohol or paraben | BHT (0.200) sodium metabisulfite (0.500) | Glycerol | EDTA | Ethanol |
| 106-108 | Phosphatidyl choline | Tween 80 | Phosphate | | BHT (0.200) sodium metabisulfite (0.500) | | EDTA | |
| 109-116 | Phosphatidyl glycerol and lysophospholipid | Brij 98 | Acetate | Benzyl alcohol, or paraben | BHT | glycerol | EDTA | Ethanol |
| 117-126 | Phosphatidyl choline and lysophospholipid | Tween 80 | Phosphate | Benzyl alcohol or paraben | BHT (0.200) sodium metabisulfite (0.500) | Glycerol | EDTA | Ethanol |
| 127-129 | Phosphatidyl choline and lysophospholipid | Brij 98 | Phosphate | Benzyl alcohol or paraben | BHT (0.200) sodium metabisulfite (0.500) | Glycerol | EDTA | Ethanol |

EP 2 437 726 B1

**Example Formulation 1**

Formulation 1 comprises sphingomyelin (brain) (47.944 mg/g) as a lipid, Tween 80 (42.056mg/g) as a surfactant, lactate buffer (pH 4), benzyl alcohol or paraben (5.000 mg/g) as an antimicrobial agent, BHT (0.200 mg/g) and sodium metabisulfite (.0500 mg/g) as antioxidants, glycerol (30.000 mg/g), EDTA (3.000 mg/g) as a chelating agent, and ethanol (30.000 mg/g).

**Example Formulation 2**

Formulation 2 comprises sphingomyelin (brain) (53.750 mg/g) as a lipid, Tween 80 (31.250 mg/g) as a surfactant, lactate (pH 4) buffer, benzyl alcohol or araben (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (15.000 mg/g).

**Example Formulation 3**

Formulation 3 comprises sphingomyelin (brain) (90.561 mg/g) as a lipid, Tween 80 (79.439 mg/g) as a surfactant, lactate (pH 4) buffer, benzyl alcohol or paraben (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 4**

Formulation 4 comprises sphingomyelin (brain) (47.944 mg/g) as a lipid, Tween 80 (42.056 mg/g) as a surfactant, lactate (pH 5) buffer, benzyl alcohol or paraben (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 5**

Formulation 5 comprises sphingomyelin lauroyl (50.607 mg/g) as a lipid, Brij 98 (44.393 mg/g) as a surfactant, acetate (pH 5) buffer, benzyl alcohol or paraben (5.000 mg/g) as an antimicrobial agent, BHT (0.200 mg/g) and sodium metabisulfite (0.500 mg/g) as antioxidants, EDTA (3.000 mg/g) as a chelating agent, and ethanol (10.000 mg/g).

**Example Formulation 6**

Formulation 6 comprises sphingomyelin lauroyl (90.561 mg/g) as a lipid, Brij 98 (79.439 mg/g) as a surfactant, acetate (pH 5) buffer, benzyl alcohol or paraben (5.000 mg/g) as as antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 7**

Formulation 7 comprises sphingomyelin lauroyl (49.276 mg/g) as a lipid, Brij 98 (79.439 mg/g) as a surfactant, acetate (pH 6.5) buffer, benzyl alcohol or paraben (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 8**

Formulation 8 comprises phosphatidyl choline and phosphatidyl glycerol (53.750 mg/g) as a lipid, Brij 98 (31.250 mg/g) as a surfactant, phosphate (pH 6.5) buffer, benzyl alcohol or paraben (5.000 mg/g) as an antimicrobial agent, HTHQ (0.200 mg/g) as an antioxidant, glycerol (30.000 mg/g), and EDTA (3.000 mg/g) as a chelating agent.

**Example Formulation 9**

Formulation 9 comprises phosphatidyl choline and phosphatidyl glycerol (90.561 mg/g) as a lipid, Brij 98 (79.439 mg/g) as a surfactant, phosphate (pH 6.5) buffer, benzyl alcohol or paraben (5.000 mg/g) as an antimicrobial agent, HTHQ (0.200 mg/g) as as antioxidant, glycerol (30.000 mg/g), EDTA (3.000 mg/g) as a chelating agent, and ethanol (30.000 mg/g).

**Example Formulation 10**

Formulation 10 comprises phosphatidyl choline and phosphatidyl glycerol (41.351mg/g) as a lipid, Brij 98 (48.649 mg/g) as a surfactant, phosphate (pH 4) buffer, benzyl alcohol or paraben (5.000 mg/g) as an antimicrobial agent, HTHQ (0.200 mg/g) as an antioxidant, glycerol (30.000 mg/g), EDTA (3.000 mg/g) as a chelating agent, and ethanol (30.000 mg/g).

**Example Formulation 11**

Formulation 11 comprises phosphatidyl choline and phosphatidyl glycerol (47.882 mg/g) as a lipid, Brij 98 (37.118 mg/g) as a surfactant, phosphate (pH 4) buffer, benzyl alcohol or paraben (5.000 mg/g) as an antimicrobial agent, HTHQ (0.200 mg/g) as an antioxidant, glycerol, EDTA (3.000 mg/g) as a chelating agent, and ethanol (30.000 mg/g).

**Example Formulation 12**

Formulation 12 comprises phosphatidyl choline and phosphatidyl glycerol (95.764 mg/g) as a lipid, Brij 98 (74.236 mg/g) as a surfactant, phosphate (pH 4) buffer, benzyl alcohol or paraben (5.000 mg/g) as an antimicrobial agent, HTHQ (0.200 mg/g) as an antioxidant, glycerol (30.000 mg/g), EDTA (3.000 mg/g) as a chelating agent, and ethanol (30.000 mg/g).

**Example Formulation 13**

Formulation 13 comprises phosphatidyl choline and phosphatidylinositol (66.676 mg/g) as a lipid, Span 20 (24.324 mg/g) as a surfactant, acetate (pH 5) buffer, benzyl alcohol or paraben (5.000 mg/g), HTHQ (0.200 mg/g) as an antioxidant, EDTA (3.000 mg/g) as a chelating agent, and ethanol (25.000 mg/g).

**Example Formulation 14**

Formulation 14 comprises phosphatidyl choline and phosphatidylinositol (62.027 mg/g) as a lipid, Span 20 (22.973 mg/g) as a surfactant, acetate (pH 5) buffer, benzyl alcohol or paraben (5.000 mg/g) as an antimicrobial agent, HTHQ (0.200 mg/g) as an antioxidant, EDTA (3.000 mg/g) as a chelating agent, and ethanol (30.000 mg/g).

**Example Formulation 15**

Formulation 15 comprises phosphatidyl choline and phosphatidylinositol (124.054 mg/g) as a lipid, Span 20 (45.946 mg/g) as a surfactant, acetate (pH 5) buffer, benzyl alcohol or paraben (5.000 mg/g) as an antimicrobial agent, HTHQ (0.200 mg/g) as an antioxidant, glycerol (30.000 mg/g), and EDTA (3.000 mg/g) as a chelating agent, and ethanol (36.510 mg/g).

**Example Formulation 16**

Formulation 16 comprises phosphatidyl choline and phosphatidylinositol (62.687 mg/g) as a lipid, Span 20 (32.313 mg/g) as a surfactant, acetate (pH 6.5) buffer, benzyl alcohol or paraben (5.000 mg/g) as an antimicrobial agent, HTHQ (0.200 mg/g) as an antioxidant, glycerol (30.000 mg/g), EDTA (3.000 mg/g) as a chelating agent.

**Example Formulation 17**

Formulation 17 comprises phosphatidyl choline and phosphatidic acid (41.853 mg/g) as a lipid, Tween 80 (43.147 mg/g) as a surfactant, phosphate (pH 6.5) buffer, benzyl alcohol or paraben (5.000 mg/g) as an antimicrobial agent, BHT (0.200 mg/g) as an antioxidant, glycerol (30.000 mg/g), EDTA (3.000 mg/g), and ethanol (30.000 mg/g).

**Example Formulation 18**

Formulation 18 comprises phosphatidyl choline and phosphatidic acid (95.764 mg/g) as a lipid, Tween 80 (74.236 mg/g) as a surfactant, phosphate (pH 6.5) buffer, benzyl alcohol or paraben (5.000 mg/g) as an antimicrobial agent, BHT (0.200 mg/g) as an antioxidant, EDTA (3.000 mg/g), and ethanol (30.000 mg/g).

**Example Formulation 19**

Formulation 19 comprises phosphatidyl choline and phosphatidic acid (47.882 mg/g) as a lipid, Brij 98 and Tween 80 (37.118 mg/g) as a surfactant, phosphate (pH 6.5) buffer, benzyl alcohol or paraben (5.000 mg/g) as an antimicrobial agent, BHT (0.200 mg/g) as an antioxidant, and EDTA (3.000 mg/g).

**Example Formulation 20**

Formulation 20 comprises phosphatidyl choline and phosphatidic acid (45.000 mg/g) as a lipid, Span 20 and Tween 80 (45.000 mg/g) as a surfactant, phosphate (pH 6.5) buffer, benzyl alcohol or paraben (5.000 mg/g) as an antimicrobial agent, BHT (0.200 mg/g) as as antioxidant, and EDTA (1.000 mg/g).

**Example Formulation 21**

Formulation 21 comprises phosphatidyl choline (31.935 mg/g) as a lipid, cremophor and Span 20 (58.065 mg/g) as a surfactant, lactate (pH 5) buffer, thimerosal (5.000 mg/g) as an antimicrobial agent, BHA (0.200 mg/g) as an antioxidant, glycerol (30.000 mg/g), EDTA (3.000 mg/g) as a chelating agent, and ethanol (15.000 mg/g).

**Example Formulation 22**

Formulation 22 comprises phosphatidyl choline (42.500 mg/g) as a lipid, cremophor and Tween 80 (42.500 mg/g) as a surfactant, lactate (pH 6.5) buffer, thimerosal (5.000 mg/g) as an antimicrobial agent, BHA (0.200 mg/g) as an antioxidant, glycerol (30.000 mg/g), and EDTA (3.000 mg/g) as a chelating agent.

**Example Formulation 23**

Formulation 23 comprises phosphatidyl choline (38.276 mg/g) as a lipid, cremophor (51.724 mg/g) as a surfactant, lactate (pH 4) buffer, thimerosal (5.000 mg/g) as an antimicrobial agent, BHA (0.200 mg/g) as an antioxidant, EDTA (3.000 mg/g) as a chelating agent, and ethanol (36.510 mg/g).

**Example Formulation 24**

Formulation 24 comprises phosphatidyl choline (42.500 mg/g) as a lipid, cremophor (42.500 mg/g) as a surfactant, lactate (pH 4) buffer, thimerosal (5.000 mg/g) as an antimicrobial agent, BHA (0.200 mg/g) as an antioxidant, EDTA (3.000 mg/g) as a chelating agent, and ethanol (15.000 mg/g).

**Example Formulation 25**

Formulation 25 comprises phosphatidyl choline (85.000 mg/g) as a lipid, cremophor (85.000 mg/g) as a surfactant, lactate (pH 4) buffer, thimerosal (5.000 mg/g) as an antimicrobial agent, BHA (0.200 mg/g) as an antioxidant, EDTA (3.000 mg/g) as a chelating agent, and ethanol (30.000 mg/g).

**Example Formulation 26**

Formulation 26 comprises phosphatidyl choline (38.276 mg/g) as a lipid, cremophor (51.276 mg/g) as a surfactant, lactate (pH 5) buffer, thimerosal (5.000 mg/g) as an antimicrobial agent, BHA (0.200 mg/g) as an antioxidant, and EDTA (1.000 mg/g) as a chelating agent.

**Example Formulation 27**

Formulation 27 comprises phosphatidyl choline (36.429 mg/g) as a lipid, cremophor (48.571 mg/g) as a surfactant, lactate (pH 5) buffer, thimerosal (5.000 mg/g) as an antimicrobial agent, BHA (0.200 mg/g) as an antioxidant, EDTA (3.000 mg/g) as a chelating agent, and ethanol (30.000 mg/g).

**Example Formulation 28**

Formulation 28 comprises phosphatidyl choline (72.299 mg/g) as a lipid, cremophor (97.701 mg/g) as a surfactant, lactate (pH 5) buffer, thimerosal (5.000 mg/g) as an antimicrobial agent, BHA (0.200 mg/g) as an antioxidant, EDTA (3.000 mg/g) as a chelating agent, and ethanol (15.000 mg/g).

**Example Formulation 29**

Formulation 29 comprises phosphatidyl ethanolamine (46.250 mg/g) as a lipid, Tween 80 (46.250 mg/g) as a surfactant, phosphate (pH 6.5) buffer, thimerosal (5.000 mg/g) as an antimicrobial agent, BHT (0.200 mg/g) and sodium metabisulfite (0.500 mg/g) as an antioxidant, EDTA (3.000 mg/g) as a chelating agent, and ethanol (20.000 mg/g).

**Example Formulation 30**

Formulation 30 comprises phosphatidyl ethanolamine (38.804 mg/g) as a lipid, Tween 80 (46.196 mg/g) as a surfactant, phosphate (pH 6.5) buffer, thimerosal (5.000 mg/g) as an antimicrobial agent, BHT (0.200 mg/g) and sodium metabisulfite (0.500 mg/g) as an antioxidant, glycerol (15.000 mg/g), EDTA (3.000 mg/g) as a chelating agent, and ethanol (30.000 mg/g).

**Example Formulation 31**

Formulation 31 comprises phosphatidyl ethanolamine (36.667 mg/g) as a lipid, Brij 98 and Tween 80 (33.333 mg/g) as a surfactant, phosphate (pH 6.5) buffer, thimerosal (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 32**

Formulation 32 comprises phosphatidyl glycerol (23.333 mg/g) as a lipid, cremophor and Brij 98 (66.667 mg/g) as a surfactant, acetate (pH 4) buffer, benzyl alcohol or paraben (5.000 mg/g) as an antimicrobial agent, BHT (0.200 mg/g) as an antioxidant, and EDTA (3.000 mg/g) as a chelating agent.

**Example Formulation 33**

Formulation 33 comprises phosphatidyl glycerol (45.833 mg/g) as a lipid, Brij 98 (41.667 mg/g) as a surfactant, acetate (pH 4) buffer, benzyl alcohol or paraben (5.000 mg/g) as an antimicrobial agent, BHT (0.200 mg/g) as an antioxidant, glycerol (30.000 mg/g), and EDTA (3.000 mg/g) as a chelating agent.

**Example Formulation 34**

Formulation 34 comprises phosphatidyl glycerol (31.957 mg/g) as a lipid, Brij 98 (38.043 mg/g) as a surfactant, acetate (pH 4) buffer, benzyl alcohol or paraben (5.000 mg/g) as as antimicrobial agent, BHT (0.200 mg/g) as an antioxidant, EDTA (3.000 mg/g) as a chelating agent, and ethanol (30.000 mg/g).

**Example Formulation 35**

Formulation 35 comprises phosphatidyl glycerol (47.143 mg/g) as a lipid, Brij 98 (42.857 mg/g) as a surfactant, acetate (pH 5) buffer, benzyl alcohol or paraben (5.000 mg/g) as an antimicrobial agent, BHT (0.200 mg/g) as an antioxidant, glycerol (30.000 mg/g), EDTA (1.000 mg/g) as a chelating agent, and ethanol (25.000 mg/g).

**Example Formulation 36**

Formulation 36 comprises phosphatidyl glycerol (96.905 mg/g) as a lipid, Brij 98 (88.095 mg/g) as a surfactant, acetate (pH 5) buffer, benzyl alcohol or paraben (5.000 mg/g) as an antimicrobial agent, BHT (0.200 mg/g) as an antioxidant, glycerol (30.000 mg/g), EDTA (3.000 mg/g) as a chelating agent, and ethanol (20.000 mg/g).

**Example Formulation 37**

Formulation 37 comprises phosphatidyl glycerol (31.957 mg/g) as a lipid, Brij 98 (38.043) as a surfactant, acetate (pH 5) buffer, benzyl alcohol or paraben (5.000 mg/g) as an antimicrobial agent, BHT (0.200 mg/g) as an antioxidant, EDTA (3.000 mg/g) as a chelating agent, and ethanol (30.000 mg/g).

**Example Formulation 38**

Formulation 38 comprises phosphatidyl ethanolamine (35.455 mg/g) as a lipid, cremophor (54.545 mg/g) as a surfactant, phosphate (pH 6.5) buffer, benzyl alcohol or paraben (5.000 mg/g) as an antimicrobial agent, BHT (0.200 mg/g) and sodium metabisulfite (0.500 mg/g) as antioxidants, glycerol (30.000 mg/g), and EDTA (3.000 mg/g) as a chelating agent.

**Example Formulation 39**

Formulation 39 comprises phosphatidyl ethanolamine (84.457 mg/g) as a lipid, cremophor (100.543 mg/g) as a surfactant, phosphate (pH 6.5) buffer, benzyl alcohol or paraben (5.000 mg/g) as an antimicrobial agent, BHT (0.200 mg/g) and sodium metabisulfite (0.500 mg/g) as antioxidants, EDTA (3.000 mg/g) as a chelating agent, and ethanol (30.000 mg/g).

**Example Formulation 40**

Formulation 40 comprises phosphatidyl ethanolamine (89.048 mg/g) as a lipid, cremophor (80.952 mg/g) as a surfactant, phosphate (pH 6.5) buffer, benzyl alcohol or paraben (5.000 mg/g), BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 41**

Formulation 41 comprises phosphatidyl glycerol (41.087 mg/g) as a lipid, Tween 80 (48.913 mg/g) as a surfactant, propionate (pH 4) buffer, benzyl alcohol or paraben (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (36.510 mg/g).

**Example Formulation 42**

Formulation 42 comprises phosphatidyl glycerol (45.280 mg/g) as a lipid, Tween 80 (39.720 mg/g) as a surfactant, propionate (pH 4) buffer, benzyl alcohol or paraben (5.000 mg/g) as an antimicrobial agent, BHT (0.200 mg/g) and sodium metabisulfite (0.500 mg/g), and EDTA (3.000 mg/g) as a chelating agent.

**Example Formulation 43**

Formulation 43 comprises phosphatidyl glycerol (107.500 mg/g) as a lipid, Tween 80 (62.500 mg/g) as a surfactant, propionate (pH 4) buffer, benzyl alcohol or paraben (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 44**

Formulation 44 comprises phosphatidyl glycerol (77.243 mg/g) as a lipid, Tween 80 (67.757 mg/g) as a surfactant, propionate (pH 4) buffer, benzyl alcohol or paraben (5.000 mg/g) as an antimicrobial agent, BHT (0.200 mg/g) and sodium metabisulfite (0.500 mg/g) as antioxidants, EDTA (3.000 mg/g) as a chelating agent, and ethanol (30.000 mg/g).

**Example Formulation 45**

Formulation 45 comprises phosphatidyl glycerol (45.280 mg/g) as a lipid, Tween 80 (39.720 mg/g) as a surfactant, propionate (pH 5) buffer, benzyl alcohol or paraben (5.000 mg/g) as an antimicrobial agent, BHT (0.200 mg/g) and sodium metabisulfite (0.500 mg/g) as antioxidants, EDTA (3.000 mg/g) as a chelating agent, and ethanol (30.000 mg/g).

**Example Formulation 46**

Formulation 46 comprises phosphatidyl glycerol (90.561 mg/g) as a lipid, Tween 80 (79.439 mg/g) as a surfactant, propionate (pH 5) buffer, benzyl alcohol or paraben (5.000 mg/g) as an antimicrobial agent, BHT (0.200 mg/g) and sodium metabisulfite (0.500 mg/g) as antioxidants, EDTA (3.000 mg/g) as a chelating agent, and ethanol (30.000 mg/g).

**Example Formulation 47**

Formulation 47 comprises phosphatidyl glycerol (47.944 mg/g) as a lipid, Tween 80 (42.056 mg/g) as a surfactant, propionate (pH 5) buffer, benzyl alcohol or paraben (5.000 mg/g) as an antimicrobial agent, BHT (0.200 mg/g) and sodium metabisulfite (0.500 mg/g) as antioxidants, EDTA (3.000 mg/g) as a chelating agent, and ethanol (10.000 mg/g).

**Example Formulation 48**

Formulation 48 comprises phosphatidyl serine (50.607 mg/g) as a lipid, Brij 98 (44.393 mg/g) as a surfactant, phosphate (pH 5.5) buffer, thimerasol (5.000 mg/g) as an antimicrobial agent, BHT (0.200 mg/g) and sodium metabisulfite (0.500 mg/g) as antioxidants, glycerol (30.000 mg/g), and EDTA (1.000 mg/g) as a chelating agent.

**Example Formulation 49**

Formulation 49 comprises phosphatidyl serine (107.500 mg/g) as a lipid, Brij 98 (62.500 mg/g) as a surfactant, phosphate (pH 5.5) buffer, thimerasol (5.000 mg/g) as an antimicrobial agent, BHT (0.200 mg/g) and sodium metabisulfite (0.500 mg/g) as antioxidants, glycerol (30.000 mg/g), and EDTA (3.000 mg/g) as a chelating agent.

**Example Formulation 50**

Formulation 50 comprises phosphatidyl serine (47.944 mg/g) as a lipid, Brij 98 (42.056 mg/g) as a surfactant, phosphate (pH 5.5) buffer, thimerasol (5.000 mg/g) as an antimicrobial agent, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 51**

Formulation 51 comprises phosphatidyl glycerol (46.364 mg/g) as a lipid, Brij 98 (38.636 mg/g) as a surfactant, acetate (pH 4) buffer, benzyl alcohol or paraben (5.000 mg/g) as an antimicrobial, BHT (0.200 mg/g) as an antioxidant, glycerol (30.000 mg/g), EDTA (3.000 mg/g) as a chelating agent, and ethanol (25.000 mg/g).

**Example Formulation 52**

Formulation 52 comprises phosphatidyl glycerol (46.364 mg/g) as a lipid, Brij 98 (38.636 mg/g) as a surfactant, acetate (pH 4) buffer, benzyl alcohol or paraben (5.000 mg/g) as an antimicrobial, BHT (0.200 mg/g) as an antioxidant, EDTA (3.000 mg/g) as a chelating agent, and ethanol (20.000 mg/g).

**Example Formulation 53**

Formulation 53 comprises phosphatidyl glycerol (46.098 mg/g) as a lipid, Brij 98 (43.902 mg/g) as a surfactant, acetate (pH 5) buffer, benzyl alcohol or paraben (5.000 mg/g) as an antimicrobial, BHT (0.200 mg/g) as an antioxidant, glycerol (15.000 mg/g), EDTA (3.000 mg/g) as a chelating agent, and ethanol (30.000 mg/g).

**Example Formulation 54**

Formulation 54 comprises phosphatidyl glycerol (43.537 mg/g) as a lipid, Brij 98 (41.463 mg/g) as a surfactant, acetate (pH 5) buffer, benzyl alcohol or paraben (5.000 mg/g) as an antimicrobial, BHT (0.200 mg/g) as an antioxidant, glycerol (30.000 mg/g), and EDTA (3.000 mg/g) as a chelating agent.

**Example Formulation 55**

Formulation 55 comprises phosphatidyl glycerol (45.000 mg/g) as a lipid, Brij 98 (45.000 mg/g) as a surfactant, acetate (pH 5) buffer, benzyl alcohol or paraben (5.000 mg/g) as an antimicrobial, BHT (0.200 mg/g) as an antioxidant, EDTA (3.000 mg/g) as a chelating agent, and ethanol (30.000 mg/g).

**Example Formulation 56**

Formulation 56 comprises phosphatidyl glycerol (59.492 mg/g) as a lipid, Brij 98 (30.508 mg/g) as a surfactant, acetate (pH 6.5) buffer, benzyl alcohol or paraben (5.000 mg/g) as an antimicrobial, BHT (0.200 mg/g) as an antioxidant, glycerol (30.000 mg/g), EDTA (3.000 mg/g) as a chelating agent, and ethanol (30.000 mg/g).

**Example Formulation 57**

Formulation 57 comprises phosphatidyl glycerol (39.054 mg/g) as a lipid, Brij 98 (45.946 mg/g) as a surfactant, acetate (pH 6.5) buffer, benzyl alcohol or paraben (5.000 mg/g) as an antimicrobial, BHT (0.200 mg/g) as an antioxidant, and EDTA (3.000 mg/g) as a chelating agent.

**Example Formulation 58**

Formulation 58 comprises phosphatidyl glycerol (35.854 mg/g) as a lipid, Brij 98 (34.146 mg/g) as a surfactant, acetate (pH 6.5) buffer, benzyl alcohol or paraben (5.000 mg/g) as an antimicrobial, BHT (0.200 mg/g) as an antioxidant, glycerol (30.000 mg/g), and EDTA (3.000 mg/g) as a chelating agent.

**Example Formulation 59**

Formulation 59 comprises phosphatidyl choline (50.000 mg/g) as a lipid, Tween 80 (40.000 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 60**

Formulation 60 comprises phosphatidyl choline (38.571 mg/g) as a lipid, Tween 80 (51.429 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g), and ethanol (30.000 mg/g).

**Example Formulation 61**

Formulation 61 comprises phosphatidyl choline (41.954 mg/g) as phospholipid, Tween 80 (50.546 mg/g) as surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g), and ethanol (30.000 mg/g).

**Example Formulation 62**

Formulation 62 comprises phosphatidyl choline (42.632 mg/g) as a lipid, Tween 80 (47.368 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 63**

Formulation 63 comprises phosphatidyl choline (46.098 mg/g) as a lipid, Tween 80 (43.902 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 64**

Formulation 64 comprises phosphatidyl choline (39.721 mg/g) as a lipid, Tween 80 (50.279 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 65**

Formulation 65 comprises phosphatidyl choline (44.198 mg/g) as a lipid, Tween 80 (50.802 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 66**

Formulation 66 comprises phosphatidyl choline (46.453 mg/g) as a lipid, Tween 80 (51.047 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 67**

Formulation 67 comprises phosphatidyl choline (51.221 mg/g) as a lipid, Tween 80 (43.779 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 68**

Formulation 68 comprises phosphatidyl choline (54.167 mg/g) as a lipid, Tween 80 (43.333 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 69**

Formulation 69 comprises phosphatidyl choline (66.440 mg/g) as a lipid, Brij 98 (23.560 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g). Example formulation 69 is an emulsion.

**Example Formulation 70**

Formulation 70 comprises phosphatidyl choline (66.440 mg/g) as a lipid, Brij 98 (23.560 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g). Example formulation 70 is a suspension.

**Example Formulation 71**

Formulation 71 comprises phosphatidyl choline (66.440 mg/g) as a lipid, Brij 98 (23.560 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 72**

Formulation 72 comprises phosphatidyl choline (40.000 mg/g) as a lipid, Tween 80 (50.000 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g). Example formulation 72 is an emulsion.

**Example Formulation 73**

Formulation 73 comprises phosphatidyl choline (40.000 mg/g) as a lipid, Tween 80 (50.000 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g). Example formulation 73 is a suspension.

**Example Formulation 74**

Formulation 74 comprises phosphatidyl choline (40.000 mg/g) as a lipid, Tween 80 (50.000 mg/g) as a surfactant, acetate (pH 5.5) buffer, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 75**

Formulation 75 comprises phosphatidyl choline (40.000 mg/g) as a lipid, Tween 80 (50.000 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 76**

Formulation 76 comprises phosphatidyl choline (40.000 mg/g) as a lipid, Brij 98 (50.000 mg/g) as a surfactant, phosphate (pH 6.5) buffer, benzalkonium chloride (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 77**

Formulation 77 comprises phosphatidyl choline (40.000 mg/g) as a lipid, Tween 80 (50.000 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 78**

Formulation 78 comprises phosphatidyl choline (66.440 mg/g) as a lipid, Brij 98 (23.560 mg/g) as a surfactant, phosphate (pH 6.5) buffer, benzalkonium chloride (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 79**

Formulation 79 comprises phosphatidyl choline (66.440 mg/g) as a lipid, Brij 98 (23.560 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 80**

Formulation 80 comprises phosphatidyl choline (40.000 mg/g) as a lipid, Tween 80 (50.000 mg/g) as a surfactant, acetate (pH 5.5) buffer, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 81**

Formulation 81 comprises phosphatidyl choline (40.000 mg/g) as a lipid, Tween 80 (50.000 mg/g) as a surfactant, acetate (pH 5.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 82**

Formulation 82 comprises phosphatidyl choline (44.444 mg/g) as a lipid, Tween 80 (55.556 mg/g) as a surfactant, acetate (pH 5.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 83**

Formulation 83 comprises phosphatidyl choline (66.440 mg/g) as a lipid, Tween 80 (23.560 mg/g) as a surfactant, acetate (pH 5.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 84**

Formulation 84 comprises phosphatidyl choline (54.000 mg/g) as a lipid, Tween 80 (36.000 mg/g) as a surfactant, acetate (pH 4) buffer, benzyl alcohol or paraben (5.000 mg/g) as an antimicrobial, BHA (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 85**

Formulation 85 comprises phosphatidyl choline (50.000 mg/g) as a lipid, Tween 80 (40.000 mg/g) as a surfactant, acetate (pH 4) buffer, benzyl alcohol or paraben (5.000 mg/g) as an antimicrobial, BHA (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 86**

Formulation 86 comprises phosphatidyl choline (48.611 mg/g) as a lipid, Tween 80 (38.889 mg/g) as a surfactant, acetate (pH 4) buffer, benzyl alcohol or paraben (5.000 mg/g) as an antimicrobial, BHA (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 87**

Formulation 87 comprises phosphatidyl choline (46.575 mg/g) as a lipid, Tween 80 (38.425 mg/g) as a surfactant, acetate (pH 4) buffer, benzyl alcohol or paraben (5.000 mg/g) as an antimicrobial, BHA (0.200 mg/g) and sodium metabisulfite (0.500 mg/g) as antioxidants, glycerol (30.000 mg/g), EDTA (3.000 mg/g) as a chelating agent, and ethanol (30.000 mg/g). Example formulation 87 is an emulsion.

**Example Formulation 88**

Formulation 88 comprises phosphatidyl choline (46.575 mg/g) as a lipid, Tween 80 (38.425 mg/g) as a surfactant, acetate (pH 4) buffer, benzyl alcohol or paraben (5.000 mg/g) as an antimicrobial, BHA (0.200 mg/g) and sodium metabisulfite (0.500 mg/g) as antioxidants, glycerol (30.000 mg/g), EDTA (3.000 mg/g) as a chelating agent, and ethanol (30.000 mg/g). Example formulation 88 is suspension.

**Example Formulation 89**

Formulation 89 comprises phosphatidyl choline (46.575 mg/g) as a lipid, Tween 80 (38.425 mg/g) as a surfactant, acetate (pH 4) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 90**

Formulation 90 comprises phosphatidyl choline (50.000 mg/g) as a lipid, Tween 80 (40.000 mg/g) as a surfactant, acetate (pH 4.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 91**

Formulation 91 comprises phosphatidyl choline (94.444 mg/g) as a lipid, Tween 80 (75.556 mg/g) as a surfactant, acetate (pH 4) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 92**

Formulation 92 comprises phosphatidyl choline (46.712 mg/g) as a lipid, Tween 80 (38.288 mg/g) as a surfactant, acetate (pH 4) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 93**

Formulation 93 comprises phosphatidyl choline (48.889 mg/g) as a lipid, Tween 80 (39.111 mg/g) as a surfactant, acetate (pH 4) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 94**

Formulation 94 comprises phosphatidyl choline (39.721 mg/g) as a lipid, Tween 80 (50.279 mg/g) as a surfactant, phosphate (pH 6.5) buffer, benzyl alcohol or paraben (5.25 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Reference Example Formulation 95**

Formulation 95 comprises phosphatidyl choline (90.000 mg/g) as a lipid, phosphate buffer (pH 6.5), benzyl alcohol or paraben as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 96**

Formulation 96 comprises phosphatidyl choline (68.700 mg/g) as a lipid, Tween 80 (8.500 mg/g) as a surfactant, phosphate (pH 7.5) buffer, BHT (0.200 mg/g) and sodium metabisulfite (0.500 mg/g) as antioxidants, benzyl alcohol or paraben (5.000 mg/g) as an antimicrobial, glycerol (30.000 mg/g), EDTA (1.000 mg/g) as a chelating agent, and ethanol (36.51 mg/g).

**Example Formulation 97**

Formulation 97 comprises phosphatidyl choline (71.460 mg/g) as a lipid, Tween 80 (4.720 mg/g) as a surfactant, phosphate (pH 7.8) buffer, BHA (0.200 mg/g) and sodium metabisulfite (0.500 mg/g) as antioxidants, benzyl alcohol or paraben (5.000 mg/g) as an antimicrobial, glycerol (50.000 mg/g), EDTA (3.000 mg/g) as a chelating agent, and ethanol (35.000 mg/g).

**Example Formulation 98**

Formulation 98 comprises phosphatidyl choline (71.460 mg/g) as a lipid, Tween 80 (4.720 mg/g) as a surfactant, phosphate (pH 7.8) buffer, BHA (0.200 mg/g) and sodium metabisulfite (0.500 mg/g) as antioxidants, benzyl alcohol or paraben (5.000 mg/g) as an antimicrobial, glycerol (15.000 mg/g), EDTA (3.000 mg/g) as a chelating agent, and ethanol (35.000 mg/g).

**Example Formulation 99**

Formulation 99 comprises phosphatidyl choline (71.460 mg/g) as a lipid, Tween 80 (4.720 mg/g) as a surfactant, phosphate (pH 7.8) buffer, BHA (0.200 mg/g) and sodium metabisulfite (0.500 mg/g) as antioxidants, glycerol (50.000 mg/g), EDTA (3.000 mg/g) as a chelating agent, and ethanol (15.000 mg/g).

**Example Formulation 100**

Formulation 100 comprises phosphatidyl choline (71.460 mg/g) as a lipid, Tween 80 (4.720 mg/g) as a surfactant, phosphate (pH 7.8) buffer, BHA (0.200 mg/g) and sodium metabisulfite (0.500 mg/g) as antioxidants, glycerol (50.000 mg/g), EDTA (3.000 mg/g) as a chelating agent, and ethanol (35.000 mg/g).

**Example Formulation 101**

Formulation 101 comprises phosphatidyl choline (46.575 mg/g) as a lipid, Tween 80 (38.425 mg/g) as a surfactant, phosphate (pH 4) buffer, BHT (0.500 mg/g) and sodium metabisulfite (0.200mg/g) as antioxidants, and EDTA (3.000 mg/g) as a chelating agent. Example formulation 101 is an emulsion.

**Example Formulation 102**

Formulation 102 comprises phosphatidyl choline (46.575 mg/g) as a lipid, Tween 80 (38.425 mg/g) as a surfactant, phosphate (pH 4) buffer, BHT (0.500 mg/g) and sodium metabisulfite (0.200mg/g) as antioxidants, and EDTA (3.000 mg/g). Example formulation 102 is a suspension.

**Example Formulation 103**

Formulation 103 comprises phosphatidyl choline (54.643 mg/g) as a lipid, Tween 80 (30.357 mg/g) as a surfactant, phosphate (pH 4) buffer, BHA (0.500 mg/g) and sodium metabisulfite (0.200mg/g) as antioxidants, and EDTA (3.000 mg/g) as a chelating agent.

**Example Formulation 104**

Formulation 104 comprises phosphatidyl choline (39.72 mg/g)as a lipid, Tween 80 (50.279 mg/g) as surfactant, phosphate (pH 6.5) buffer, benzyl alcohol or paraben (5.00 mg/g) as an antimicrobial, BHT (0.200 mg/g) and sodium metabisulfite (0.500 mg/g) as antioxidants, glycerol (30.000 mg/g) as emollient, EDTA (3.000 mg/g) as the chelating agent, and ethanol (30.000 mg/g).

**Reference Example Formulation 105**

Formulation 105 comprises phosphatidyl choline (90.00 mg/g) as a lipid, phosphate (pH 6.5) buffer, benzyl alcohol or paraben as antimicrobial (5.000 mg/s), BHT (0.200 mg/g) and sodium metabisulfite (0.500 mg/g) as antioxidants, glycerol (30.000 mg/g) as emollient, EDTA (3.000 mg/g) as the chelating agent, and ethanol (30.000 mg/g).

**Example Formulation 106**

Formulation 106 comprises phosphatidyl choline (46.57 mg/g) as a lipid, Tween 80 (38.425 mg/g) as a surfactant, phosphate (pH 4) buffer, BHT (0.500 mg/g) and sodium metabisulfite (0.200mg/g) as antioxidants, and EDTA (3.000 mg/g) as the chelating agent. Formulation 106 is formulated as an emulsion.

**Example Formulation 107**

Formulation 107 comprises phosphatidyl choline (46.57 mg/g) as a lipid, Tween 80 (38.425 mg/g) as a surfactant, phosphate (pH 4) buffer, BHT (0.500 mg/g) and sodium metabisulfite (0.200mg/g) as antioxidants, and EDTA (3.000 mg/g) as the chelating agent. Formulation 107 as a suspension.

**Example Formulation 108**

Formulation 108 comprises phosphatidyl choline (54.64 mg/g) as a lipid, Tween 80 (30.357 mg/g) as a surfactant, phosphate (pH 4) buffer, BHA (0.500 mg/g) and sodium metabisulfite (0.200mg/g) as antioxidants, EDTA (3.000 mg/g) as the chelating agent.

**Example Formulation 109**

Formulation 109 comprises phosphatidyl glycerol and lysophospholipid (46.364 mg/g) as a lipid, Brij 98 (38.636 mg/g) as a surfactant, acetate (pH 4) buffer, benzyl alcohol or paraben (5.000 mg/g) as an antimicrobial, BHT (0.200 mg/g) as an antioxidant, glycerol (30.000 mg/g), EDTA (3.000 mg/g) as a chelating agent, and ethanol (25.000 mg/g).

**Example Formulation 110**

Formulation 110 comprises phosphatidyl glycerol and lysophospholipid (46.364 mg/g) as a lipid, Brij 98 (38.636 mg/g) as a surfactant, acetate (pH 4) buffer, benzyl alcohol or paraben (5.000 mg/g) as an antimicrobial, BHT (0.200 mg/g) as an antioxidant, EDTA (3.000 mg/g) as a chelating agent, and ethanol (20.000 mg/g).

**Example Formulation 111**

Formulation 111 comprises phosphatidyl glycerol and lysophospholipid (46.098 mg/g) as a lipid, Brij 98 (43.902 mg/g) as a surfactant, acetate (pH 5) buffer, benzyl alcohol or paraben (5.000 mg/g) as an antimicrobial, BHT (0.200 mg/g) as an antioxidant, glycerol (15.000 mg/g), EDTA (3.000 mg/g) as a chelating agent, and ethanol (30.000 mg/g).

**Example Formulation 112**

Formulation 112 comprises phosphatidyl glycerol and lysophospholipid (43.537 mg/g) as a lipid, Brij 98 (41.463 mg/g) as a surfactant, acetate (pH 5) buffer, benzyl alcohol or paraben (5.000 mg/g) as an antimicrobial, BHT (0.200 mg/g) as an antioxidant, glycerol (30.000 mg/g), and EDTA (3.000 mg/g) as a chelating agent.

**Example Formulation 113**

Formulation 113 comprises phosphatidyl glycerol and lysophospholipid (45.000 mg/g) as a lipid, Brij 98 (45.000 mg/g) as a surfactant, acetate (pH 5) buffer, benzyl alcohol or paraben (5.000 mg/g) as an antimicrobial, BHT (0.200 mg/g) as an antioxidant, EDTA (3.000 mg/g) as a chelating agent, and ethanol (30.000 mg/g).

**Example Formulation 114**

Formulation 114 comprises phosphatidyl glycerol and lysophospholipid (59.492 mg/g) as a lipid, Brij 98 (30.508 mg/g) as a surfactant, acetate (pH 6.5) buffer, benzyl alcohol or paraben (5.000 mg/g) as an antimicrobial, BHT (0.200 mg/g) as an antioxidant, glycerol (30.000 mg/g), EDTA (3.000 mg/g) as a chelating agent, and ethanol (30.000 mg/g).

**Example Formulation 115**

Formulation 115 comprises phosphatidyl glycerol and lysophospholipid (39.054 mg/g) as a lipid, Brij 98 (45.946 mg/g) as a surfactant, acetate (pH 6.5) buffer, benzyl alcohol or paraben (5.000 mg/g) as an antimicrobial, BHT (0.200 mg/g) as an antioxidant, and EDTA (3.000 mg/g) as a chelating agent.

**Example Formulation 116**

Formulation 116 comprises phosphatidyl glycerol and lysophospholipid (35.854 mg/g) as a lipid, Brij 98 (34.146 mg/g) as a surfactant, acetate (pH 6.5) buffer, benzyl alcohol or paraben (5.000 mg/g) as an antimicrobial, BHT (0.200 mg/g) as an antioxidant, glycerol (30.000 mg/g), and EDTA (3.000 mg/g) as a chelating agent.

**Example Formulation 117**

Formulation 117 comprises phosphatidyl choline and lysophospholipid (50.000 mg/g) as a lipid, Tween 80 (40.000 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 118**

Formulation 118 comprises phosphatidyl choline and lysophospho lipid (38.571 mg/g) as a lipid, Tween 80 (51.429 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g), and ethanol (30.000 mg/g).

**Example Formulation 119**

Formulation 119 comprises phosphatidyl choline and lysophospholipid (41.954 mg/g) as phospholipid, Tween 80 (50.546 mg/g) as surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g), and ethanol (30.000 mg/g).

**Example Formulation 120**

Formulation 120 comprises phosphatidyl choline and lysophospholipid (42.632 mg/g) as a lipid, Tween 80 (47.368 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 121**

Formulation 121 comprises phosphatidyl choline and lysophospholipid (46.098 mg/g) as a lipid, Tween 80 (43.902 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 122**

Formulation 122 comprises phosphatidyl choline and lysophospholipid (39.721 mg/g) as a lipid, Tween 80 (50.279 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 123**

Formulation 123 comprises phosphatidyl choline and lysophospholipid (44.198 mg/g) as a lipid, Tween 80 (50.802 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 124**

Formulation 124 comprises phosphatidyl choline and lysophospholipid (46.453 mg/g) as a lipid, Tween 80 (51.047 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 125**

Formulation 125 comprises phosphatidyl choline and lysophospholipid (51.221 mg/g) as a lipid, Tween 80 (43.779 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 126**

Formulation 126 comprises phosphatidyl choline (54.167 mg/g) as a lipid, Tween 80 (43.333 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

**Example Formulation 127**

Formulation 127 comprises phosphatidyl choline and lysophospholipid (66.440 mg/g) as a lipid, Brij 98 (23.560 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g). Example formulation 69 is an emulsion.

**Example Formulation 128**

Formulation 128 comprises phosphatidyl choline and lysophospholipid (66.440 mg/g) as a lipid, Brij 98 (23.560 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g). Example formulation 70 is a suspension.

**Example Formulation 129**

Formulation 129 comprises phosphatidyl choline and lysophospholipid (66.440 mg/g) as a lipid, Brij 98 (23.560 mg/g) as a surfactant, phosphate (pH 6.5) buffer, benzyl alcohol or paraben (5.000 mg/g) as an antimicrobial, BHT (0.200 mg/g) and sodium metabisulfite (0.500 mg/g) as antioxidants, glycerol (30.000 mg/g), EDTA (3.000 mg/g) as a chelating agent, and ethanol (30.000 mg/g).

It will be understood that the exact amounts of the components of the formula may be adjusted slightly without departing from the scope of the invention. For example, in each of the above formulations, the amount antimicrobial be anywhere from about 1 mg/g to about 15 mg/g, or about 5 m/g to about 12 mg/g, or 5.25 mg/g, 6, mg/6, 7 mg/g, 8 mg/g, 9 mg/g, 10 mg/g, or 10.25 mg/g. Furthermore, the antimicrobial can be a combination of ingredients, for example benzyl alcohol and parabenes (e.g., ethyl and/or propyl).

Example Formulations 1 through 129 may also optionally include thickeners such as pectin, xanthan gum, HPMC gel, methylcellulose or carbopol.

**Claims**

1.  A vesicular formulation for use in the treatment of pain associated with osteoarthritis comprising one or more phospholipids and one or more non-ionic surfactants and being free of any non-lipid non-surfactant pharmaceutically active agent, wherein the formulation is topically applied.

2.  A vesicular formulation as claimed in claim 1 for use in the treatment of pain, including deep tissue pain.

3.  A vesicular formulation as claimed in any preceding claim, wherein the molar ratio of phospholipid to surfactant is in the range of from about 1:3 to about 30:1, preferably about 1:1 to about 30:1, and more preferably about 2:1 to about 20:1.

4. A vesicular formulation as claimed in any preceding claim, wherein the formulation contains 2.0-10.0% wt phospholipid.

5. A vesicular formulation as claimed in any preceding claim, wherein said one or more phospholipids include phosphatidylcholine.

6. A vesicular formulation as claimed in any preceding claim, wherein the formulation contains 0.2-5.0% wt surfactant.

7. A vesicular formulation as claimed in any preceding claim, wherein the formulation comprises one or more non-ionic surfactants selected from poloxyethylene sorbitans, polyhydroxyethylene stearates and polyhydroxyethylene laurylethers.

8. A vesicular formulation as claimed in any preceding claim, wherein said one or more surfactants comprise polysorbate 80.

9. A vesicular formulation for use in the treatment of pain associated with osteoarthritis which consists essentially of one or more phospholipids and one or more non-ionic surfactants in a pharmaceutically acceptable carrier, wherein the formulation is topically applied.

10. A vesicular formulation as claimed in claim 10, wherein the molar ratio of phospholipid to surfactant is in the range of from about 1:3 to about 30:1, preferably about 1:1 to about 30:1, and more preferably about 2:1 to about 20:1.

11. A vesicular formulation as claimed in claim 10 or claim 11, wherein said one or more phospholipids include phosphatidylcholine.

12. A vesicular formulation as claimed in claim 10, claim 11 or claim 12, wherein the formulation comprises one or more non-ionic surfactants selected from polyoxyethylene sorbitans, polyhydroxyethylene stearates and polyhydroxyethylene laurylethers.

13. A vesicular formulation as claimed in any of claims 10 to 13, wherein said formulation further includes one or more buffers, chelators, humectants, lubricants, antioxidants, preservatives, microbiocides, antimicrobials, emollients, co-solvents or thickeners.

**Patentansprüche**

1. Vesikuläre Formulierung zur Verwendung in der Behandlung von mit Osteoarthritis zusammenhängendem Schmerz, umfassend ein oder mehrere Phospholipide und ein oder mehrere nichtionische Tenside, und die frei von jedwedem pharmazeutischem Wirkstoff ist, der kein Lipid bzw. Tensid ist, wobei die Formulierung topisch aufgetragen wird.

2. Vesikuläre Formulierung nach Anspruch 1 zur Verwendung in der Behandlung von Schmerz einschließlich tiefem Gewebeschmerz.

3. Vesikuläre Formulierung nach einem der vorhergehenden Ansprüche, wobei das Molverhältnis von Phospholipid zu Tensid im Bereich von etwa 1:3 bis etwa 30:1, vorzugsweise etwa 1:1 bis etwa 30:1 und bevorzugter etwa 2:1 bis etwa 20:1 ist.

4. Vesikuläre Formulierung nach einem der vorhergehenden Ansprüche, wobei die Formulierung 2,0 bis 10,0 Gew.% Phospholipid enthält.

5. Vesikuläre Formulierung nach einem der vorhergehenden Ansprüche, wobei das eine oder die mehreren Phospholipide Phosphatidylcholin einschließen.

6. Vesikuläre Formulierung nach einem der vorhergehenden Ansprüche, wobei die Formulierung 0,2 bis 5,0 Gew.% Tensid enthält.

7. Vesikuläre Formulierung nach einem der vorhergehenden Ansprüche, wobei die Formulierung ein oder mehrere nichtionische Tenside ausgewählt aus Polyoxyethylensorbitanen, Polyhydroxyethylenstearaten und Polyhydroxyethylenlaurylethern umfasst.

**8.** Vesikuläre Formulierung nach einem der vorhergehenden Ansprüche, wobei das eine oder die mehreren Tenside Polysorbat 80 einschließen.

**9.** Vesikuläre Formulierung zur Verwendung in der Behandlung von mit Osteoarthritis zusammenhängendem Schmerz, die im Wesentlichen aus einem oder mehreren Phospholipiden und einem oder mehreren nichtionischen Tensiden in einem pharmazeutisch annehmbaren Träger besteht, wobei die Formulierung topisch aufgebracht wird.

**10.** Vesikuläre Formulierung nach Anspruch 10, wobei das Molverhältnis von Phospholipid zu Tensid im Bereich von etwa 1:3 bis etwa 30:1, vorzugsweise etwa 1:1 bis etwa 30:1 und bevorzugter etwa 2:1 bis etwa 20:1 liegt.

**11.** Vesikuläre Formulierung nach Anspruch 10 oder Anspruch 11, wobei das eine oder die mehreren Phospholipide Phosphatidylcholin einschließen.

**12.** Vesikuläre Formulierung nach Anspruch 10, Anspruch 11 oder Anspruch 12, wobei die Formulierung ein oder mehrere nichtionische Tenside ausgewählt aus Polyoxyethylensorbitanen, Polyhydroxyethylenstearaten und Polyhydroxyethylenlaurylethern umfasst.

**13.** Vesikuläre Formulierung nach einem der Ansprüche 10 bis 13, wobei die Formulierung ferner einen oder mehrere Puffer, Chelatbildner, Feuchthaltemittel, Schmiermittel, Antioxidantien, Konservierungsmittel, Mikrobiozide, antimikrobielle Mittel, Emollienzien, Colösungsmittel oder Verdickungsmittel einschließt.

**Revendications**

**1.** Formulation vésiculaire pour utilisation dans le traitement de la douleur associée à l'arthrose comprenant un ou plusieurs phospholipides et un ou plusieurs tensioactifs non ioniques et étant exempte de tout agent pharmaceutiquement actif non tensioactif, non lipidique, la formulation étant appliquée par voie topique.

**2.** Formulation vésiculaire selon la revendication 1 pour utilisation dans le traitement de la douleur, comprenant une douleur tissulaire profonde.

**3.** Formulation vésiculaire selon l'une quelconque des revendications précédentes, dans laquelle le rapport molaire du phospholipide au tensioactif est dans la plage d'environ 1:3 à environ 30:1, de préférence d'environ 1:1 à environ 30:1, et plus préférablement d'environ 2:1 à environ 20:1.

**4.** Formulation vésiculaire selon l'une quelconque des revendications précédentes, la formulation contenant de 2,0 à 10,0 % en poids de phospholipide.

**5.** Formulation vésiculaire selon l'une quelconque des revendications précédentes, dans laquelle lesdits un ou plusieurs phospholipides comprennent la phosphatidylcholine.

**6.** Formulation vésiculaire selon l'une quelconque des revendications précédentes, la formulation contenant de 0,2 à 5,0 % en poids de tensioactif.

**7.** Formulation vésiculaire selon l'une quelconque des revendications précédentes, la formulation comprenant un ou plusieurs tensioactifs non ioniques choisis parmi des sorbitanes de polyoxyéthylène, des stéarates de polyhydroxyéthylène et des éthers lauryliques de polyhydroxyéthylène.

**8.** Formulation vésiculaire selon l'une quelconque des revendications précédentes, dans laquelle lesdits un ou plusieurs tensioactifs comprennent le polysorbate 80.

**9.** Formulation vésiculaire pour utilisation dans le traitement de la douleur associée à l'arthrose qui est essentiellement constituée d'un ou plusieurs phospholipides et d'un ou plusieurs tensioactifs non ioniques dans un véhicule pharmaceutiquement acceptable, la formulation étant appliquée par voie topique.

**10.** Formulation vésiculaire selon la revendication 10, dans laquelle le rapport molaire du phospholipide au tensioactif est dans la plage d'environ 1:3 à environ 30:1, de préférence d'environ 1:1 à environ 30:1, et plus préférablement d'environ 2:1 à environ 20:1.

11. Formulation vésiculaire selon la revendication 10 ou la revendication 11, dans laquelle lesdits un ou plusieurs phospholipides comprennent la phosphatidylcholine.

12. Formulation vésiculaire selon la revendication 10, la revendication 11 ou la revendication 12, la formulation comprenant un ou plusieurs tensioactifs non ioniques choisis parmi les sorbitanes polyoxyéthyléniques, les stéarates polyhydroxyéthyléniques et les éthers lauryliques polyhydroxyéthyléniques.

13. Formulation vésiculaire selon l'une quelconque des revendications 10 à 13, ladite formulation comprenant en outre un ou plusieurs tampons, chélateurs, humectants, lubrifiants, antioxydants, conservateurs, microbiocides, antimicrobiens, émollients, cosolvants ou épaississants.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6165500 A, Cevc
- US 20040071767 A, Cevc
- US 20040105881 A, Cevc
- EP 0475160 A1
- US 20020012680 A1
- EP 0475160 A
- WO 9817255 A

**Non-patent literature cited in the description**

- **BARTHEL et al.** Randomized controlled trial of disclofenac sodium gel in knee osteoarthritis. *Seminars in Arthritis and Rheeumatism,* 01 December 2009, vol. 39 (3), 203-212
- **CEVC.** *Biochim. Biophys. Acta,* 2002, vol. 1564, 21-30